(19) Europäisches Patentamt / European Patent Office / Office européen des brevets

(11) **EP 3 958 728 B1**

(12) **FASCICULE DE BREVET EUROPEEN**

(45) Date de publication et mention de la délivrance du brevet:
**18.09.2024   Bulletin 2024/38**

(21) Numéro de dépôt: **20719643.7**

(22) Date de dépôt: **22.04.2020**

(51) Classification Internationale des Brevets (IPC):
***A61B 3/12*** *(2006.01)*      ***A61B 3/10*** *(2006.01)*
***G06T 7/00*** *(2017.01)*

(52) Classification Coopérative des Brevets (CPC):
**A61B 3/12; A61B 3/1025;** G06T 2207/30041

(86) Numéro de dépôt international:
**PCT/EP2020/061147**

(87) Numéro de publication internationale:
**WO 2020/216763 (29.10.2020 Gazette 2020/44)**

(54) **PROCEDE DE TOMOGRAPHIE EN INCOHERENCE OPTIQUE EN OPHTALMOLOGIE**

VERFAHREN ZUR OPTISCHEN INKOHÄRENZTOMOGRAFIE IN DER OPHTHALMOLOGIE

METHOD FOR OPTICAL INCOHERENCE TOMOGRAPHY IN OPHTHALMOLOGY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **23.04.2019   FR 1904271**

(43) Date de publication de la demande:
**02.03.2022   Bulletin 2022/09**

(73) Titulaires:
• **Quantel Medical**
  **63808 Cournon d'Auvergne Cedex (FR)**
• **Office National d'Etudes et de Recherches Aérospatiales (ONERA)**
  **91120 Palaiseau (FR)**

(72) Inventeurs:
• **BARACAL DE MECE, Pedro**
  **92200 NEUILLY S/SEINE (FR)**
• **MEIMON, Serge**
  **92322 CHATILLON (FR)**
• **PETIT, Cyril**
  **91370 VERRIERES LE BUISSON (FR)**
• **PUREUR, David**
  **63808 COURNON-D'AUVERGNE CEDEX (FR)**
• **WASSMER, Benjamin**
  **63808 COURNON-D'AUVERGNE CEDEX (FR)**

(74) Mandataire: **Regimbeau**
  **20, rue de Chazelles**
  **75847 Paris Cedex 17 (FR)**

(56) Documents cités:
**US-A1- 2013 063 698      US-A1- 2014 268 049**

**Description**

DOMAINE TECHNIQUE

La présente invention appartient au domaine de l'ophtalmologie. Plus précisément, l'invention concerne un procédé de tomographie en incohérence optique permettant une imagerie in vivo temps réel de la rétine, un système d'imagerie ophtalmique, et un système de photocoagulation laser d'une rétine.

ETAT DE LA TECHNIQUE

L'oedème maculaire est une affection rétinienne assez fréquente se caractérisant par un d'un oedème de la région centrale de la rétine (macula) lié à des anomalies des petits vaisseaux sanguins, entraînant une baisse de l'acuité visuelle. L'oedème maculaire est la principale manifestation de la rétinopathie diabétique, qui est la cause principale de cécité avant 55 ans. Le traitement standard de ces oedèmes maculaires consiste à réaliser une photocoagulation de ces vaisseaux dans la zone centrale de la rétine à l'aide d'un laser. D'autres maladies peuvent également bénéficier de la photocoagulation de la macula, comme les occlusions veineuses rétiniennes.
Lors d'une photocoagulation, la cible thérapeutique du laser peut se situer au niveau de deux couches :

- la couche des vaisseaux sanguins rétiniens, dans le cas d'un oedème localisé. Dans ce cas, l'oedème est causé par une dilatation vasculaire appelée macroanévrysme sur un vaisseau rétinien identifié, et l'on procède à une photocoagulation directe du vaisseau impliqué ;
- l'épithélium pigmentaire rétinien, dans le cas d'un oedème diffus. Une solution consiste alors à stimuler la fonction d'absorption de l'épithélium pigmentaire, en le photocoagulant avec des impacts répétés.

Ces deux couches de la rétine sont séparées d'une centaine de microns, et sont entourés d'autres couches correspondant à des tissus fonctionnels qu'il faut s'efforcer de préserver, sous peine de provoquer une baisse de vision définitive.
Jusqu'à présent, le chirurgien opérant le laser ne disposait que d'une visualisation bidimensionnelle de la rétine à la lampe à fente, vue de face, sur laquelle le chirurgien s'appuie pour localiser le point d'impact laser en surface de la rétine, et doser la puissance du laser : il est recommandé d'appliquer le laser jusqu'à observer un discret blanchiment à la lampe à fente. Il apparaît donc que dans les procédures chirurgicales actuelles, la focalisation et le dosage du laser sont très empiriques. De fait, les procédures de traitement standard sont assez peu reproductibles d'un praticien à l'autre.
En outre, pendant l'opération, l'oeil est plus ou moins stabilisé avec un verre de contact maintenu sur l'oeil, qui ne filtre que très partiellement les continuels mouvements involontaires de fixation de l'oeil. Il en résulte que l'impact laser peut ne pas correspondre à la position visée sur l'image de fond d'oeil obtenue par la lampe à fente.
Pour pallier ce manque de précision dans la localisation de l'impact laser, les systèmes actuels sont configurés pour générer un impact laser de grande taille, afin de s'assurer que la photocoagulation recouvre entièrement la zone à traiter. La grande taille de l'impact laser est obtenue par la délivrance d'un faisceau laser large, avec un diamètre latéral de la tache focale sur la rétine allant de 100 $\mu$m à 500 $\mu$m, et présentant une faible ouverture optique, avec une extension longitudinale (en profondeur) de la tache focale sur la rétine d'environ 300 $\mu$m.
Par ailleurs, un impact laser de grande taille permet également de pallier différentes aberrations oculaires réduisant la précision de la localisation de l'impact laser. En effet, un oeil humain réel n'est pas strictement stigmatique, c'est-à-dire que l'image d'un point n'est pas un point rigoureusement net. Ces aberrations oculaires peuvent être statiques, les exemples les plus courants étant les défauts de vision corrigés par les lunettes (myopie, hypermétropie, astigmatisme, etc.). Les aberrations peuvent également être dynamiques, par exemple causées par des micro-accommodations du cristallin, l'écoulement du film lacrymal, et les mouvements oculaires. Ces aberrations se traduisent par une fonction d'étalement du point ("Point Spread Function" ou PSF en anglais) qui s'éloigne de celle d'un oeil parfait théorique, et qui varie rapidement dans le temps.
La grande taille des impacts laser actuels n'est pas adaptée à la taille des zones à traiter. A titre d'exemple, les macro-anévrysmes présentent des tailles variant typiquement entre 100 $\mu$m et 300 $\mu$m, alors même que l'impact laser présente un diamètre d'environ 300 $\mu$m, sans compter la diffusion thermique se produisant autour de la zone d'impact laser. Il en résulte qu'apparaissent des lésions des tissus sains avoisinant la zone à traiter. Or, la couche des vaisseaux sanguins rétiniens et l'épithélium pigmentaire rétinien sont entourés de tissus fonctionnels dont l'altération peut entraîner une baisse de vision définitive.
Il est donc nécessaire de disposer d'un moyen d'imagerie efficace, offrant une grande précision et une grande réactivité afin de pouvoir rendre compte des mouvements de l'oeil.
Les ophtalmoscope peuvent être classés en deux catégories :

- l'ophtalmoscope à champ plein (ou FIO pour "flood-illumination ophtalmoscope" en anglais), où la rétine est illuminée en champ plein, et un imageur acquiert une image de la lumière réfléchie qui ressort de l'oeil;
- l'ophtalmoscope à balayage laser (ou SLO pour l'anglais "scanning laser ophtalmoscope"), où la rétine est illuminée par une source ponctuelle et un imageur acquiert une image de la lumière réfléchie qui ressort de l'oeil, l'image étant acquise au cours du balayage laser de la surface à imager.

Le SLO peut bénéficier d'un sectionnement optique en plaçant un écran percé d'un petit trou au plan focal de l'imageur, approche désignée comme confocale. L'écran percé permet de rejeter physiquement les photons en provenance des plans hors foyer.

Chaque type d'ophtalmoscope peut être utilisé suivant deux configurations de détection en fonction des propriétés de la lumière utilisées : en incohérence ou en cohérence. La détection en cohérence se fait principalement selon la technique de tomographie en cohérence optique, ou OCT pour l'anglais "optical cohérence tomography". Cette technique consiste à produire des interférences entre la lumière réfléchie par la rétine et une partie de la lumière d'illumination utilisée comme référence. Grâce aux motifs d'interférence générés, la différence de temps de propagation entre la rétine et le faisceau de référence peut être déterminée, permettant une résolution axiale de quelques micromètres.

Toutefois, dans les systèmes OCT, des perturbations oculaires comme par exemple les mouvements des yeux ou des aberrations optiques, affectent la qualité de l'image rétinienne. Alors que les effets des mouvements des yeux (flou et distorsions) peuvent être minimisés par un post-traitement, les aberrations oculaires sont évitées par l'utilisation d'un système avec une faible ouverture optique, typiquement entre 1 mm et 2 mm. A l'exception de la résolution axiale qui ne dépend pas de l'ouverture optique, cette petite ouverture optique limite la résolution dans les autres directions, et certaines caractéristiques rétiniennes ne sont pas imagées convenablement.

En particulier, un système OCT est faiblement sensible au défocus (correspondant à un changement d'accommodation de l'oeil), et est fortement sensible aux mouvements axiaux de la rétine (pulsation) et de l'oeil. Comme le positionnement axial d'un laser thérapeutique est donné par un défocus pur, l'OCT se révèle impropre à être utilisée pour déterminer et contrôler le défocus à être appliqué sur le laser thérapeutique afin de bien positionner axialement l'impact laser sur la cible thérapeutique et ainsi éviter d'endommager les tissus rétiniens sains adjacents à la zone d'impact désirée du laser.

Des systèmes d'imagerie tomographique des différents couches rétinales sont divulgués dans les documents US 2014/268049 et US 2013/063698.

PRESENTATION DE L'INVENTION

L'invention a pour but de permettre la construction d'une représentation tomographique dans laquelle chaque caractéristique présente une position axiale connue. A cet effet, il est proposé un procédé d'imagerie ophtalmique d'une partie à imager d'un oeil au moyen d'un système d'imagerie ophtalmique, ledit système d'imagerie ophtalmique comprenant :

- une source lumineuse adaptée pour émettre un faisceau lumineux d'éclairage,
- un imageur adapté pour acquérir une image d'un faisceau lumineux renvoyé,
- un système optique adapté pour la propagation de faisceaux lumineux, le système optique comprenant une partie commune s'étendant jusqu'à une interface face à l'oeil, une première branche reliant la source lumineuse à la partie commune, et une seconde branche reliant l'imageur à la partie commune,

le procédé comprenant les étapes suivantes :

a) éclairage de la partie à imager de l'oeil par un faisceau lumineux d'éclairage émis par la source lumineuse, et acquisition par l'imageur d'une série d'images de la zone à imager correspondants à une pluralité de plans focaux objet différents distribués le long d'un axe objet, l'acquisition comprenant une modification d'un paramètre de variation de la seconde branche du système optique entre chaque image acquise,

b) application d'un filtrage passe-haut en fréquences spatiales sur chacune des images de la série d'image pour obtenir une série d'images filtrées,

c) pour chaque image filtrée, détermination d'au moins une valeur de métrique représentative de l'énergie d'une zone d'intérêt de chaque image filtrée, et

d) combinaison de valeurs d'une métrique représentative de l'énergie de différentes images filtrées, chacune associée avec des coordonnées axiales représentatives de la distribution des plans focaux objet le long de l'axe objet, pour obtenir une représentation tomographique.

Le procédé est avantageusement complété par les caractéristiques suivantes, prises seules ou en une quelconque de leur combinaison techniquement possible :

- les coordonnées axiales correspondent à des valeurs représentatives d'un paramètre de variation de la seconde branche des images acquises correspondant aux images filtrées ;
- la modification de la seconde branche du système optique entre chaque image acquise comprend une translation de l'imageur, et/ou une modification d'un élément optique présent dans la seconde branche du système optique ;
- la série d'images de la rétine comprend au moins 20 images acquises sans modification de la première branche et de la partie commune du système optique ;
- l'acquisition de la série d'images est effectuée en moins de 5 secondes;
- la représentation tomographique est une représentation de type A qui fait correspondre une métrique représentative de l'énergie pour une pluralité de coordonnées axiales, ou la représentation tomographique est une représentation de type B qui fait correspondre une métrique représentative de l'énergie pour une pluralité de coordonnées axiales et pour une pluralité de coordonnées dans une direction perpendiculaire à l'axe objet ;
- des images acquises successives sont combinées entre elles pour résulter en une image combinée dont les valeurs de pixels correspondent à une combinaison des valeurs des pixels des images acquises successives ;
- au moins trois images acquises successives sont combinées entre elles, et la combinaison des valeurs des pixels des images acquises successives consiste en une moyenne de ces valeurs des pixels des images acquises successives ;
- la métrique représentative de l'énergie est une énergie de fréquence spatiale, une variance spatiale de l'image, une énergie laplacienne de l'image, ou une fonction de Brenner de l'image ;
- le procédé comprend en outre une étape e) dans laquelle une valeur représentative d'un paramètre de variation de la seconde branche correspondant à un maximum local de la métrique d'énergie est déterminé à partir de la représentation, et la seconde branche est ensuite réglée selon cette valeur représentative du paramètre de variation de sorte que le plan focal image ait une position correspondant à une coordonnée axiale du maximum local de la métrique d'énergie, et au moins une image est acquise.

L'invention concerne également un système d'imagerie ophtalmique comprenant :

- une source lumineuse adaptée pour émettre un faisceau lumineux d'éclairage,
- un imageur adapté pour acquérir une image d'un faisceau lumineux renvoyé,
- un système optique adapté pour la propagation de faisceaux lumineux, le système optique comprenant une partie commune s'étendant jusqu'à une interface face à l'oeil, une première branche reliant la source lumineuse à la partie commune, et une seconde branche reliant l'imageur à la partie commune,

le système d'imagerie ophtalmique étant configuré pour:

a) éclairer la partie à imager de l'oeil par un faisceau lumineux d'éclairage émis par la source lumineuse, et acquérir par l'imageur une série d'images de la zone à imager correspondants à une pluralité de plans focaux objet différents distribués le long d'un axe objet, l'acquisition comprenant une modification de la seconde branche du système optique entre chaque image acquise,
b) appliquer un filtrage passe-haut en fréquences spatiales sur chacune des images de la série d'image pour obtenir une série d'images filtrées,
c) pour chaque image filtrée, déterminer au moins une métrique représentative de l'énergie d'une zone d'intérêt de chaque image filtrée, et
d) combiner des valeurs d'une métrique représentative de l'énergie de différentes images filtrées, chacune associée avec des coordonnées axiales représentatives de la distribution des plans focaux objet le long de l'axe objet, et obtenir une représentation tomographique,

le système d'imagerie ophtalmique étant configuré pour mettre en oeuvre le procédé selon l'invention.

De préférence, le système de photocoagulation laser d'une rétine, comprend :

- le système d'imagerie ophtalmique décrit,
- un laser de photocoagulation configuré pour émettre dans une direction d'émission un faisceau laser dans le système optique,

le système de photocoagulation laser comprenant un organe de commande du laser de photocoagulation, l'organe de commande étant configuré pour déterminer, à partir de la représentation tomographique, une valeur représentative d'un paramètre de variation de la seconde branche correspondant à une coordonnée axiale d'une cible selon l'axe objet dans la représentation tomographique, et pour commander le laser de photocoagulation au moyen d'une commande de profondeur dérivée de cette valeur représentative d'un paramètre de variation. De préférence, le laser est mobile en translation dans sa direction d'émission et la commande de profondeur est une commande de déplacement du laser.

PRESENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :

- la figure 1 montre schématiquement une vue simplifiée d'un exemple de système d'imagerie selon un mode de réalisation possible de l'invention ;
- la figure 2 montre une vue de couches d'une rétine, avec l'origine des photons recueillis par l'imageur selon un mode de réalisation possible de l'invention ;
- la figure 3 est un schéma illustrant la superposition de plans imagés le long de l'axe objet dans une image acquise selon un mode de réalisation possible de l'invention ;
- la figure 4a est la 200ème image acquise d'un exemple d'une série d'images acquises selon un mode de réalisation possible de l'invention ;
- la figure 4b est la 20ème image filtrée résultant de la combinaison et du filtrage de la série d'images de la figure 4a selon un mode de réalisation possible de l'invention ;
- la figure 5a est la 600ème image acquise d'un exemple d'une série d'images acquises selon un mode de réalisation possible de l'invention ;
- la figure 5b est la 60ème image filtrée résultant de la combinaison et du filtrage de la série d'images de la figure 5a selon un mode de réalisation possible de l'invention ;
- la figure 6 est un exemple de représentation tomographique de type A selon un mode de réalisation possible de l'invention ;
- la figure 7 est une image acquise au plan focal objet correspondant à la coordonnée 190 $\mu$m sur la représentation tomographique de la figure 6 ;
- la figure 8 est une image acquise au plan focal objet correspondant à la coordonnée 30 $\mu$m sur la représentation tomographique de la figure 6 ;
- la figure 9 est un schéma illustrant une première étape de construction d'une représentation tomographique de type B selon un mode de réalisation possible de l'invention,
- la figure 10 est un schéma illustrant le résultat de la première étape de construction d'une représentation tomographique de type B selon un mode de réalisation possible de l'invention ;
- la figure 11 est un exemple de représentation tomographique de type B selon un mode de réalisation possible de l'invention ;
- la figure 12 est un exemple d'image OCT de la même zone imagée de la rétine que la figure 11 ;
- la figure 13 présente ainsi un exemple plus détaillé de système d'imagerie selon un mode de réalisation possible de l'invention.

DESCRIPTION DETAILLEE

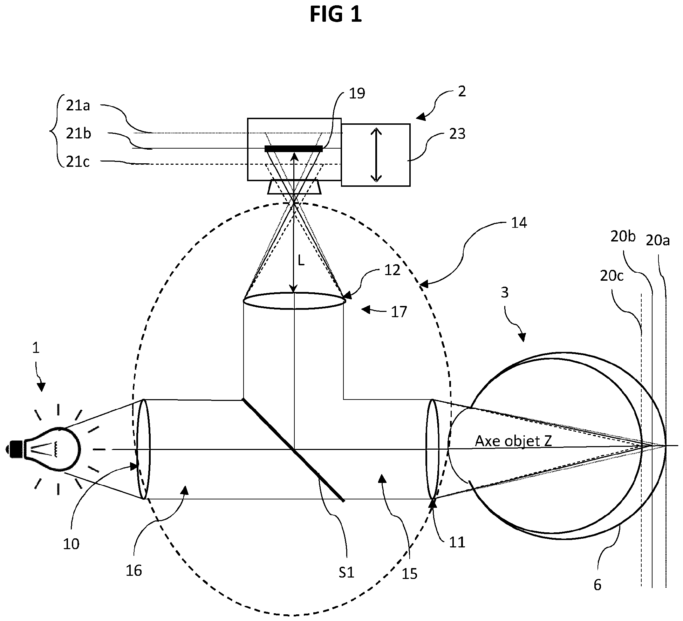

En référence à la figure 1, le système d'imagerie ophtalmique comprend une source lumineuse 1 adaptée pour émettre un faisceau lumineux d'éclairage et un imageur 2 configuré pour acquérir une image d'un faisceau lumineux renvoyé par une partie à imager d'un oeil 3 éclairé par le faisceau lumineux d'éclairage. Entre la source lumineuse 1, l'imageur 2 et la partie à imager de l'oeil 3 se trouve un chemin optique dans lequel se propagent les faisceaux lumineux entre la source lumineuse 1, l'imageur 2 et la partie à imager de l'oeil 3.

Le chemin optique est constitué des différents composant traversés par les faisceaux lumineux. Le chemin optique comprend ainsi une partie de l'oeil 3 que traverse le faisceau lumineux d'éclairage pour atteindre la partie à imager et que traverse le faisceau lumineux renvoyé pour ressortir de l'oeil 3 à partir de la partie à imager. Par exemple, lorsque la partie à imager est une rétine, le chemin optique comprend la cornée, le cristallin et le corps vitré. Le chemin optique comprend également plusieurs éléments optiques permettant de réaliser différentes fonctions lors de la propagation des faisceaux lumineux, tels que des lentilles qui permettent de faire converger ou diverger les faisceaux lumineux les traversant ou des miroirs pour orienter les faisceaux. Ces éléments optiques forment un système optique 14 comprenant :

- une entrée 10 face à la source lumineuse 1 par laquelle pénètre le faisceau lumineux d'éclairage émis par la source lumineuse 1,

- une interface 11 face à l'oeil 3 par laquelle sort le faisceau lumineux d'éclairage pour atteindre l'oeil 3 et par laquelle le faisceau lumineux d'image en provenance de l'oeil entre dans le système optique,
- une sortie 12 face à l'imageur 2, par laquelle sort le faisceau lumineux d'image pour atteindre l'imageur 2.

Le système optique 14 peut ainsi être décomposé en une partie commune 15 s'étendant jusqu'à l'interface 11 face à l'oeil 3, une première branche 16 reliant la source lumineuse 1 à la partie commune 15, et une seconde branche 17 reliant l'imageur 2 à la partie commune 15. Typiquement, un séparatrice S1 est disposée dans le chemin optique permet de séparer la première branche 16 et/ou la seconde branche 17 par rapport à la partie commune 15 du chemin lumineux. Dans la description, une séparatrice, ou "beam splitter" en anglais, permet de laisser passer une partie d'un rayon lumineux incident tout en réfléchissant une autre partie dans une autre direction, et peut être de tout type, comme par exemple un miroir semi-réfléchissant. Dans l'exemple de la figure 1, une seule séparatrice S1 est disposée, afin que le faisceau lumineux renvoyé par l'oeil 3 parcourant la partie commune 15 du chemin optique soit dévié vers la seconde branche 17 à destination de l'imageur 2. Il est bien sûr possible de prévoir plus de branches, et plus de séparatrices S1, en fonction du nombre d'éléments du système qui doivent utiliser le chemin optique 14.

La source lumineuse 1 est configurée pour éclairer la rétine de l'oeil 3 afin que l'imageur 2 puisse acquérir une image de la rétine de l'oeil 3. La source lumineuse 1 peut émettre dans toutes longueurs d'onde susceptible d'être utilisée pour mettre en évidence des caractéristiques de l'oeil 3. Toutefois, la source lumineuse émet de préférence de la lumière à une ou plusieurs longueurs d'onde uniquement comprise entre 800 nm et 900 nm. Contrairement à l'OCT, le présent procédé ne repose pas sur l'exploitation d'interférences lumineuses. La source lumineuse 1 n'a donc pas besoin d'être cohérente, et peut être incohérente.

L'imageur 2 comprend une optique et un capteur d'image 19 qui peut être de tout type susceptible de permettre d'imager la rétine de l'oeil 3, et par exemple être un capteur CCD. De préférence, l'imageur 2 est capable d'acquérir des images à une fréquence supérieure à 100 Hz, et de préférence à une fréquence supérieure ou égale à 150 Hz. L'imageur 2 peut être configuré pour faire des acquisitions plein champ, c'est-à-dire acquérant à chaque prise de vue toute l'image transverse envisagée pour le plan focal image (par exemple de dimension 4° par 4° sur la surface rétinienne). Alternativement, l'imageur 2 peut être en configuration dite confocale, avec un écran percé d'un trou au plan focal de l'imageur. Dans cette alternative, un scanner est utilisé pour balayer transversalement la position de l'image du trou confocal. Toutefois, si cette configuration permet d'augmenter le contraste et d'améliorer le rapport signal sur bruit, elle a l'inconvénient d'être plus complexe à mettre en oeuvre, et surtout de diminuer fortement la vitesse d'acquisition, pouvant ainsi engendrer des distorsions entre des parties d'images ou entre des images en raison des mouvements de l'oeil.

Les propriétés optiques du chemin optique définissent un plan focal objet 20 dans l'oeil 3 et un plan focal image 21 au niveau de l'imageur 2, le plan focal image 21 étant le plan conjugué du plan focal objet 20. Le plan focal objet 20 désigne la zone de l'oeil 3 en provenance de laquelle les photons seront focalisé au niveau du plan focal image 21 de l'imageur 2, et produiront par conséquence une image nette.

Les positions du plan focal objet 20 et du plan focal image 21 dépendent des caractéristiques optiques du chemin optique, c'est-à-dire des caractéristiques optiques de ses différents composants, ainsi que de leur arrangement spatial. Toutefois, les caractéristiques optiques de la partie de l'oeil 3 traversé par les faisceaux lumineux ne sont pas connues en détail, et sont variables au cours du temps. En effet, outre les mouvements oculaires, un oeil 3 présente en permanence des micro-accommodations du cristallin par modification de sa vergence, et diverses autres variation dynamiques, qui empêche de connaître a priori la position exacte du plan focal image. De plus, la rétine humaine est un volume complexe composé de couches hypo-réflective et hyper-réflective. Dans une épaisseur d'environ 150 $\mu$m au centre de la fovéa, de 300 $\mu$m en bordure de la fovéa, et de 200 $\mu$m ailleurs, se trouve notamment au fond la couche des photorécepteurs, en contact avec la couche d'épithélium pigmentaire rétinien, et d'autres couches telles que les couches plexiformes internes et externes, etc.. Un mouvement latéral de l'oeil 3 peut ainsi induire un déplacement du plan focal image, en particulier dans la mesure où trois éléments particulièrement réfléchissants sont disposés dans différentes couches de la rétine : les photorécepteurs, les vaisseaux sanguins et les fibres nerveuses.

Si les caractéristiques optiques de la partie de l'oeil 3 traversé par les faisceaux lumineux ne peuvent être commandés, il est possible en revanche de modifier les caractéristiques optiques du système optique 14 afin de déplacer le plan focal objet 20 le long de l'axe objet Z. Plus précisément, une modification de la seconde branche 17 du système optique 14 permet de déplacer le plan focal image 21 et son conjugué le plan focal objet 20. La seconde branche 17 du système optique 14 est configurée pour être modifiée afin de déplacer le plan focal objet 20 selon l'axe objet Z.

Plusieurs possibilités existent pour modifier la seconde branche 17 afin d'obtenir le déplacement du plan focal objet 20 le long de l'axe objet Z. De préférence, la modification de la seconde branche 17 du système optique 14 peut être effectuée par une modification d'une distance L dans cette seconde branche 2 entre le capteur 19 de l'imageur 2 et la sortie 12 qui fait face à l'imageur, par exemple constitué par une lentille ou tout autre composant optique. Cette modification de la distance L peut en particulier être obtenue par une translation de l'imageur 2. A cet effet, l'imageur 2 peut être mobile en translation contrôlée dans la direction de propagation de la lumière que reçoit l'imageur 2 en provenance du système optique 14. Pour ce faire, l'imageur 2 peut être monté sur une platine motorisée qui permet, en fonction de commandes reçues, de déplacer en translation l'imageur 2 grâce à un moteur 23. Il est également possible que la modification de la seconde branche 17 du système optique 14 soit effectuée par une modification d'un élément optique présent dans cette seconde branche 17. Il est par exemple possible de faire varier la surface d'un miroir déformable, ou de modifier la forme d'une lentille liquide, par exemple constituant la sortie 12 du système optique 14.

Préalablement à la mise en oeuvre du procédé, il est préférable de dilater la pupille du patient et de paralyser son accommodation. Il est par exemple possible d'utiliser à cet effet une goutte de tropicamide et une goutte de phenylphrine 10%. La largeur de la pupille obtenue est de préférence supérieure à 5 mm. De fait, une pupille dilatée permet d'obtenir une plus grande ouverture numérique oculaire, permettant que la lumière incidente soit mieux collimatée et focalise mieux sur la rétine. La résolution latérale (perpendiculaire à la direction de propagation du faisceau, en x, y) de la lumière incidente sur la rétine est proportionnelle (pour un oeil parfait à ouverture circulaire) à l'inverse de l'ouverture numérique oculaire, tandis que l'extension en profondeur est proportionnelle au carré de l'inverse de l'ouverture numérique oculaire. Il en résulte que la forte ouverture numérique oculaire permet de limiter la déformation (et notamment l'élargissement) du faisceau lumineux d'éclairage au niveau de la tache focale sur la rétine, et donc de mieux confiner l'éclairage. De plus, en dilatant la pupille, le système d'imagerie est moins impacté par la diffraction des faisceaux lumineux. Par ailleurs, la paralysie de l'accommodation permet de limiter les effets de la dynamique de la mise au point (défocus) sur l'évolution temporelle de la résolution axiale.

Le procédé commence par l'éclairage de la rétine par un faisceau lumineux d'éclairage émis par la source lumineuse 1. La source lumineuse 1 émet le faisceau lumineux d'éclairage qui traverse l'entrée 10, se propage à travers la première branche 16, traverse la séparatrice S1, se propage à travers la partie commune 15 du système optique 14, traverse l'interface 11, puis atteint l'oeil 3. Le faisceau lumineux d'éclairage traverse la pupille, le corps vitré, et atteint la rétine 6. La lumière rencontre les différents tissus constituant la rétine 6, organisés en couches rétiniennes à différentes profondeurs.

Au contact de ces tissus, la lumière peut être réfléchie, diffusée, transmise ou absorbée. Les photons réfléchis et diffusés forment alors un faisceau lumineux renvoyé qui retourne dans le système optique en passant par le corps vitré, la pupille, et l'interface 11, puis à travers la partie commune 15, et qui est partiellement dévié par la séparatrice SI le long de la seconde branche 17 en direction de la sortie 12 et finalement de l'imageur 2, où les photons sont recueillis pour former une image.

Comme indiqué plus haut, tous les photons recueillis par l'imageur 2 ne donnent pas une image nette. Seuls les photons en provenance du plan focal objet 20 conjugué au plan focal image 21 où se trouve le capteur 19 de l'imageur 2 forment une image nette. En déplaçant la position du plan focal objet 20, il est donc possible d'imager nettement différentes zones de l'oeil 3 distribuées à différentes profondeurs le long de l'axe objet 20.

Le procédé prévoit donc l'acquisition par l'imageur 2 d'une série d'images d'une zone à imager de l'oeil 3, les images correspondant à une pluralité de plans focaux objet 20 différents distribués le long de l'axe objet Z. La série d'images comprend au moins 20 images, et de préférence au moins 50 images, et de préférence encore au moins 100 images. L'éclairage de la zone à imager de l'oeil 3 par le faisceau lumineux d'éclairage peut être maintenu pendant toute la durée d'acquisition, ou peut être intermittent et synchronisé avec chaque prise de vue pendant l'acquisition.

Entre chaque image, le système d'imagerie ophtalmique est modifié pour que le plan focal objet 20 soit déplacé le long de l'axe objet z. A cet effet, l'acquisition comprend une modification de la seconde branche 17 du système optique 14 entre chaque image acquise, de préférence sans modification de la première branche 16 ou de la partie commune 15. Comme indiqué plus haut, la modification de la seconde branche 17 du système optique 14 entre chaque image acquise peut par exemple comprendre une translation de l'imageur 2, et/ou une modification d'un élément optique présent dans la seconde branche 17 du système optique 14.

Lors de l'acquisition, la distribution des plans focaux objet 20 s'étend dans la zone à imager de l'oeil 3 le long de l'axe objet Z sur une distance supérieure à moins 50 $\mu$m, et de préférence supérieure à 100 $\mu$m. Il est même possible que cette distance soit supérieure à 300 $\mu$m, afin de couvrir toute l'épaisseur typique de la rétine. De préférence, deux plans focaux objet adjacents correspondant à deux images successives sont distants de moins de 20 $\mu$m, et de préférence de moins de 10 $\mu$m, et de préférence encore de moins de 5 $\mu$m le long de l'axe objet Z.

Le déplacement du plan focal objet 20 peut être effectué de manière continue, par exemple par la translation continue de l'imageur 2, et de préférence à une vitesse constante. Cela permet une rapidité accrue et évite d'éventuelles saccades. Cependant, il est alors nécessaire de réduire la durée d'exposition pour chaque image afin d'éviter la présence d'artéfacts liés au mouvement, ce qui induit un rapport signal sur bruit moins favorable. Il est également possible d'opérer en pas à pas le déplacement de l'imageur 2. On évite alors les défauts optiques résultants du déplacement de l'imageur 2 lors de l'exposition, au détriment toutefois de la vitesse d'acquisition. Il est par ailleurs possible d'augmenter la durée d'exposition pour chaque image afin d'améliorer le rapport signal sur bruit. La vitesse de translation du plan focal objet 20 est par exemple comprise entre 50 et 200 $\mu$m/s, comme par exemple de 100 $\mu$m/s dans l'axe objet Z.

L'imageur 2 acquiert des images avec une fréquence comprise entre 50 Hz et 400 Hz, et de préférence entre 100 Hz et 300 Hz. Le temps d'exposition du capteur 19 pour chaque image est un compromis entre un temps suffisamment long afin d'obtenir sur l'image acquise un rapport signal sur bruit satisfaisant, mais pas trop long afin de ne pas être affecté d'un flou de bougé résultant de mouvements de l'oeil 3 pendant le temps d'exposition du capteur 19. Ainsi, le temps d'exposition du capteur 19 est de préférence inférieur à 10 ms. Afin d'éviter de trop importants mouvements de l'oeil 3, l'acquisition de la série d'images est de préférence effectuée en moins de 5 secondes, et de préférence encore en moins de 3 secondes.

La relation entre le déplacement de l'imageur 2 et le déplacement correspondant du plan focal objet dans la zone à imager de l'oeil 3 dépend des caractéristiques du système d'imagerie ophtalmique. La relation peut par exemple être une relation quadratique. Le grandissement optique du système d'imagerie ophtalmique doit également être pris en compte. Par exemple, avec un agrandissement par un facteur 100, un déplacement du plan focal objet de 1 $\mu$m le long de l'axe objet Z nécessite un déplacement de 100 $\mu$m de l'imageur 2. Un grandissement important est donc avantageux, car il est plus facile de contrôler un déplacement de 100 $\mu$m de l'imageur 2 que de contrôler un déplacement de 1 $\mu$m de l'imageur 2.

A chaque image acquise est associée une valeur représentative d'un paramètre de variation de la seconde branche 17 du système optique 14 correspondant à la valeur prise par ce paramètre lors de l'acquisition de ladite image. Par exemple, lors d'une translation de l'imageur 2, à chaque image peut correspondre une position de l'imageur 2 le long de l'axe image au moment où cette image est acquise. Cette position est alors associée à ladite image, éventuellement sous une forme transformée. Il en va de même pour le focus variable lorsque la seconde branche 17 du système optique 14 est modifiée au moyen d'une déformation d'un miroir déformable ou d'une lentille. A cet effet, le système comprend un capteur configuré pour déterminer une valeur représentative d'un paramètre de variation de la seconde branche 17 du système optique 14, le système étant configuré pour synchroniser l'acquisition d'une image avec la mesure de la valeur du paramètre de variation. A titre d'exemple, en cas de translation de l'imageur 2, un capteur de position est commandé pour acquérir une mesure de la position de la platine supportant l'imageur 2 à chaque acquisition d'image.

Plusieurs positions de plans focaux au cours de l'acquisition d'images ont été représentés sur la figure 1, avec en tant qu'exemple non limitatif une modification de la seconde branche 17 du système optique 14 par la modification de la distance L entre le capteur 19 de l'imageur 2 et la sortie 12. Au début de l'acquisition par l'imageur 2 de la série d'images, le système d'imagerie est configuré de sorte qu'un premier plan focal objet 2a se trouve au niveau du fond de la rétine 6 de l'oeil 3. L'imageur 2, dont le capteur 19 se trouve au plan focal image 21a, acquiert une image. Puis l'imageur 2 est déplacé le long de l'axe image pour réduire la distance L entre le capteur 19 et la sortie 12. Le plan focal image 21b est donc déplacé, de même que son conjugué le plan focal objet 20b. Le plan focal objet 20b se situe désormais moins profond dans la rétine 6. L'imageur 2 acquiert une autre image. Puis l'imageur 2 est déplacé le long de l'axe image pour réduire encore la distance L entre le capteur 19 et la sortie 12. Le plan focal image 21c est donc déplacé, de même que son conjugué le plan focal objet 20c. Le plan focal objet 20c se situe désormais à la surface de la rétine 6, et l'imageur 2 acquiert une image. Il est à noter qu'en fonction de la configuration du système d'imagerie ophtalmique, à l'inverse de ce qui décrit ci-dessus un raccourcissement de la distance L entre la sortie 12 et le capteur 19 (et donc un rapprochement du plan focal image 21) peut engendrer un éloignement du plan focal objet 20 le long de l'axe objet Z par rapport à l'interface 11.

Les images acquises, normalement plus nombreuses que les trois de l'exemple simplifié ci-dessus, forment une série d'images acquises, spatialement ordonnées par les positions respectives du plan focal objet 20 lors de leurs acquisitions. De fait, l'ordre des images de la série d'image correspond à l'ordre des positions du plan focal objet 20 le long de l'axe objet Z. Le contenu des images de la série d'image reflète donc la structure tridimensionnelle de la zone imagée de l'oeil 3.

Il existe une relation entre la modification apportée à la seconde branche 17 du système optique 14 et le déplacement du plan focal objet 21 le long de l'axe objet Z, puisque c'est la modification apportée à la seconde branche 17 qui cause le déplacement du plan focal objet 21 le long de l'axe objet Z. Il est donc possible d'associer des coordonnées axiales, au moins relatives, à chaque image, ces coordonnées axiales correspondant directement à des valeurs représentatives d'un paramètre de variation de la seconde branche 17 et étant représentatives de de la distribution des plans focaux objet le long de l'axe image Z. Par exemple, si la modification de la seconde branche 17 consiste à déplacer d'une distance $d$ l'imageur 2 entre l'acquisition de deux images, et qu'il existe un rapport de 1 à 10 sur le déplacement du plan focal objet 21 sur l'axe objet Z, les deux images successives représenteront des plans imagés de la zone à imager distants de $d/10$, et leurs coordonnées axiales seront distantes de $d/10$.

Il est à noter que les positions exactes des plans focaux objet 20a, 20b, 20c le long de l'axe objet Z n'ont pas à être connues exactement avant l'acquisition d'images. En effet, dans la mesure où les différents plans focaux objet 20a, 20b, 20c sont distribués le long de l'axe objet Z, il est possible de déterminer a posteriori les profondeurs imagées. Ainsi, le plan focal objet 20b peut correspondre au fond de la rétine 6, le plan focal objet 20a étant par exemple trop profond.

Les valeurs de pixels d'une image de la série d'images peuvent être ensuite modifiées par les valeurs de pixels d'une autre image de la série d'images, afin d'améliorer le rapport signal sur bruit, ladite image et ladite autre image étant des images successives de la série d'images. Plus précisément, les images peuvent résulter de la moyenne glissante des images. Il serait possible de conserver le même nombre d'images acquises, en modifiant les valeurs de chaque image avec les images la précédant ou la suivant dans la série. De préférence toutefois, des images successives sont combinées entre elles, de sorte à réduire le nombre d'images de la série d'image. A cet effet, il est possible de procéder à un suréchantillonnage axial (selon l'axe objet Z), en acquérant des images successives à des plans focaux objet 20 distants entre eux d'une distance inférieure à la profondeur de champ, et de combiner de telles images ensembles, afin d'améliorer le rapport signal sur bruit. Par exemple, avec une profondeur de champ de 20 $\mu$m, et si les images sont acquises avec des plans focaux objet 20 distants de 2 $\mu$m le long de l'axe objet Z, il est possible de combiner ensemble dix images successives qui présentent un recouvrement spatial en raison de la superposition de leurs profondeurs de champ. On obtient alors dans cet exemple une série d'images avec cinq fois moins d'images qu'initialement. L'image obtenue peut correspondre à la moyenne des images combinées, ou plus précisément les valeurs des pixels de l'image obtenue peuvent résulter de la moyenne des valeurs des pixels correspondants des images combinées. Cette moyenne peut éventuellement être pondérée, par exemple avec un poids plus fort pour les images centrales de l'ensemble des images combinées.

Puisqu'à chaque image acquise est associée une valeur représentative d'un paramètre de variation de la seconde branche 17, il est aisé de conserver une telle valeur associée à chaque image combinée. Typiquement, la valeur représentative du paramètre de variation associée à une image centrale sont conservées pour l'image combinée. La valeur représentative du paramètre de variation de l'image résultant de la combinaison peut également correspondre à la moyenne des valeurs représentatives du paramètre de variation associées à chacune des images qui ont été combinées.

Chacune des images acquises, éventuellement combinées entre elles, résultent de la collecte des photons lors de l'exposition de l'imageur 2 au moment de la prise de vue. Idéalement, seule la lumière en provenance de la position du plan focal objet 20 à l'instant de l'acquisition de l'image devrait atteindre l'imageur, générant une image nette de la zone imagée où est situé le plan focal objet 21. Cependant, dans la réalité, les photons qui sont rétrodiffusés en provenance des plans hors foyer atteignent également l'imageur.

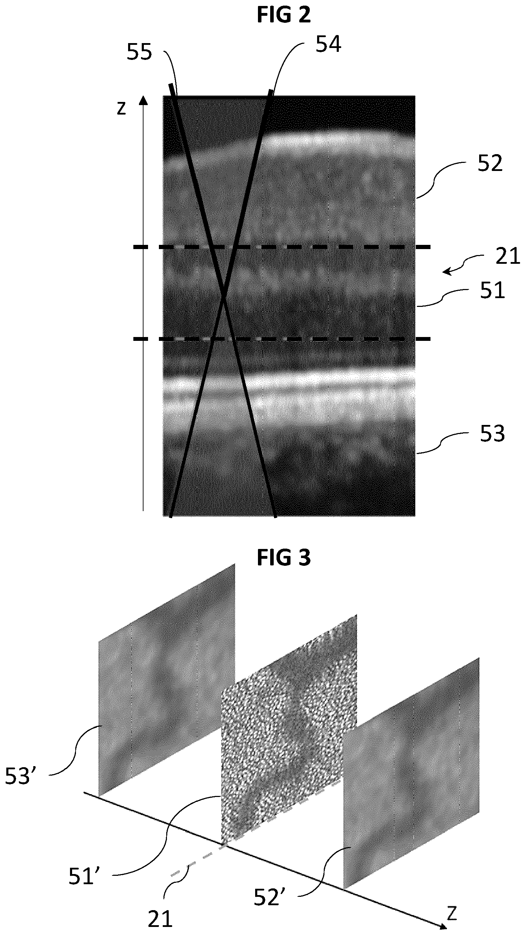

La **figure 2** montre une vue de couches de la rétine 6, la direction verticale correspondant à l'axe de la profondeur de la rétine 6 et donc à l'axe objet z. La première zone 51 délimitée par des tirets blancs correspond à la zone imagée comprise dans la profondeur de champ du plan focal objet 20. La deuxième zone 52 est au-delà du plan focal objet 20, tandis que la troisième zone 53 est en-deçà du plan focal objet 20.

Les deux lignes blanches 54, 55 délimitent les origines des photons collectés par l'imageur 2 dans cette configuration. Les photons originaires de la première zone 51 proviennent de localisations très proches, et forment donc des caractéristiques nettes sur l'image acquise. Cette première zone 51 produit donc des composantes de basses, moyennes et hautes fréquences spatiales, qui dépendent de la structure de la rétine dans cette première zone 51. Les photons originaires de la deuxième zone 52 et de la troisième zone 53 sont hors foyer, et proviennent de localisations diverses dans ces zones. Ils ne peuvent donc former des caractéristiques nettes. La deuxième zone 52 et la troisième zone 53 produisent donc seulement des composantes de basses et moyennes fréquences spatiales. Seuls les photons en provenance du voisinage du plan focal image peuvent générer les hautes fréquences spatiales.

La **figure 3** illustre schématiquement la conséquence de cet effet. Chaque image acquise correspond à la superposition de plusieurs plans situés à des profondeurs différentes le long de l'axe objet Z. Au niveau du plan focal objet 20 correspond un premier plan 51' formés par les photons en provenance de la première zone 51, sur lesquels les caractéristiques sont nettes. L'image comprend cependant d'autres plans 52', 53' correspondant aux zones 52, 53 hors foyers, pour lesquelles les caractéristiques sont floues.

Dans la mesure où ces différents plans 51', 52', 53' sont fusionnés dans l'image acquise, l'image rend compte d'une zone à imager plus large que sa profondeur de champ, d'une manière floue toutefois. En éliminant par filtrage les éléments de basses et moyennes fréquences spatiales, il est possible de limiter l'influence des plans 52', 53'. L'image devient alors restreinte aux photons originaires de la première zone 51 correspondant à la position du plan focal objet 20.

On applique ainsi un sectionnement optique numérique, le sectionnement optique désignant la capacité du système d'imagerie à rejeter les photons en provenance des plans hors foyer.

Pour mettre en oeuvre ce sectionnement optique numérique, il est procédé à l'application d'un filtrage passe-haut en fréquences spatiales sur chacune des images de la série d'image pour obtenir une série d'images filtrées. Le filtrage passe-haut vise à supprimer dans les images acquises les composantes de basses et moyennes fréquences spatiales, c'est-à-dire à filtrer les photons hors foyer collectés par l'imageur 2. La fréquence de coupure de ce filtre passe-haut est choisie pour être supérieure à la taille moyenne des structures d'intérêt de la rétine (vaisseaux sanguins par exemple), qui présentent une taille inférieure à 50 $\mu$m. Avec un filtre de Butterworth normalisé d'ordre 2, cela correspond à une fréquence spatiale de coupure comprise entre 0,04 et 0,1, et de préférence comprise entre 0,06 et 0,09.

Il est également possible de mettre en oeuvre un filtre passe-bas en fréquences spatiales pour retirer les très hautes fréquences spatiales, typiquement supérieures à la fréquence spatiale des photorécepteurs du capteur 19, qui n'apparaissent que comme du bruit dans les images. On peut par exemple choisir une fréquence de coupure supérieure à 1 pour un filtre de Butterworth normalisé d'ordre 2, voire supérieure à 0,8 pour un filtre de Butterworth normalisé d'ordre 2 si le rapport signal sur bruit n'est pas satisfaisant. Le filtre passe-haut et le filtre passe-bas peuvent être combiné en un filtre passe-bande.

Lors de l'acquisition de la série d'images, il est possible que l'oeil 3 bouge entre deux prises de vue, engendrant entre des images successives un décalage transversalement à l'axe objet Z. Il est donc possible de mettre en oeuvre un recalage des images, c'est-à-dire une translation et/ou une rotation d'une image par rapport à une autre. Les paramètres du recalage peuvent être déterminés au moyen d'un suivi du mouvement de la zone à imager (typiquement la rétine), par exemple avec un imageur dédié acquérant une vidéo dont l'analyse déterminerait les mouvements à compenser, et donc les paramètres. Il est également possible de procéder à un recalage au moyen d'un algorithme mettant en oeuvre une corrélation entre images filtrées successives.

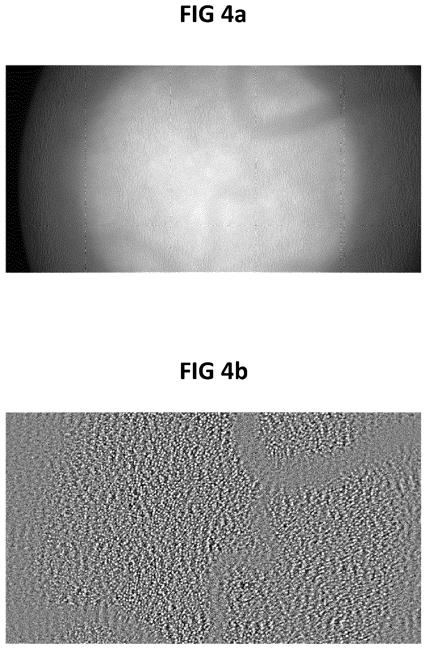

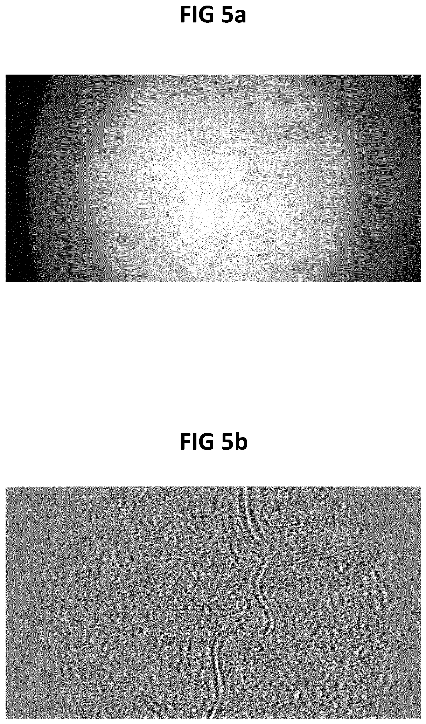

Après application au moins du filtre passe-haut (éventuellement sous la forme d'un filtre passe-bande), suivi éventuellement du recalage, on obtient alors une série d'images filtrées. La **figure 4a** et la **figure 5a** montrent des exemples d'images acquises d'une série d'images de 900 images. La **figure 4a** montre l'image numéro 200 de la série d'image, et la **figure 5a** montre l'image numéro 600. Les images de la série d'images sont combinées 10 images par 10 images, résultant en une série de 90 images combinées remplaçant les 900 images acquises. Plus précisément, les valeurs de pixels d'une image combinée résultent de la moyenne de 10 images acquises successives de la série d'images. Puis un filtrage passe-bande est appliqué.

La **figure 4b** montre l'image 20 de la série d'images correspondant à l'image numéro 200 illustrée par la **figure 4a,** et la **figure 5b** montre l'image numéro 60 de la série de 90 images combinées correspondant à l'image 600 illustrée par la **figure 5b.** Il est aisé de constater qu'alors que les images acquises étaient très floues, les images finales sont très nettes. Comme expliqué plus haut, le filtrage a retiré les influences des plans hors foyer qui engendraient du flou. Alors que les images acquises représentaient une superposition de plans le long de l'axe objet Z, les images finales ne représentent plus que la zone imagée de l'oeil au niveau du plan focal objet. Les traitements appliqués permettent donc une discrimination spatiale le long de l'axe objet Z. Par ailleurs, la présence des cellules de la rétine est désormais mise en évidence, alors que ces cellules n'étaient pas visibles dans les images acquises telles que celles des figures 4a et 5a.

Il est à noter que le filtrage ne modifie pas l'association de chaque image avec des valeurs représentatives d'un paramètre de variation de la seconde branche 17, et donc des coordonnées axiales représentatives de la distribution des plans focaux objet le long de l'axe objet Z.

Une fois obtenue la série d'images filtrée, au moins une valeur d'une métrique représentative de l'énergie d'une zone d'intérêt de chaque image filtrée est déterminée. Plusieurs métriques peuvent être utilisées en tant que métrique représentative de l'énergie. Il est par exemple possible de déterminer une énergie de fréquence spatiale. En notant p(i,j) le niveau de gris d'un pixel aux coordonnées i,j d'une image filtrée de dimensions N x M, l'énergie de fréquence spatiale E peut par exemple s'exprimer comme :

[Math 1]

$$E = \sum_{i=1}^{N} \sum_{j=1}^{M} abs\left[\widetilde{I(\iota,j)}\right]^2$$

avec la transformée de Fourier de l'image filtrée :

[Math 2]

$$\widetilde{I(\iota,j)} = \sum_{n=0}^{N-1} \sum_{m=0}^{M-1} I(n,m) e^{-i2\pi\left(\frac{in}{N} - \frac{jm}{M}\right)}$$

Une autre métrique possible représentative de l'énergie est la variance spatiale de l'image, qui peut par exemple s'exprimer comme :

[Math 3]

$$E = \frac{1}{N-1}\frac{1}{M-1}\sum_{i=1}^{N}\sum_{j=1}^{M}[p(i,j) - \bar{p}]^2$$

Une autre métrique possible représentative de l'énergie est l'énergie laplacienne de l'image, qui peut par exemple s'exprimer comme :

[Math 4]

$$E = \sum_{i=1}^{N}\sum_{j=1}^{M}[p(i+1,j) + p(i-1,j) + p(i,j+1) + p(i,j-1) - 4 \times p(i,j)]^2$$

Une autre métrique possible représentative de l'énergie est la fonction de Brenner de l'image, qui peut par exemple s'exprimer comme :

[Math 5]

$$E = \sum_{i=1}^{N}\sum_{j=1}^{M}[p(i,j) - p(i-2,j)]^2$$

Les différentes possibilités de métrique ci-dessus ont été données pour l'image filtrée entière en tant que sous-zone. Il est toutefois possible si souhaité de restreindre la sous-zone à une partie de l'image filtrée. Par ailleurs, il est possible d'utiliser des métriques différentes pour une même image, bien que de préférence la même métrique soit utilisée.

Des valeurs de métrique représentative de l'énergie de différentes images filtrées ainsi déterminées sont ensuite combinées pour obtenir une représentation tomographique. Si plusieurs métriques sont utilisées, il s'agit d'une combinaison de valeurs métriques représentatives de l'énergie. Comme chaque image filtrée est associée avec des valeurs représentatives d'un paramètre de variation de la seconde branche correspondant à des coordonnées axiales représentatives de la distribution des plans focaux objet le long de l'axe, les valeurs de métrique représentative de l'énergie de ces images filtrées le sont également. L'obtention de la représentation tomographique consiste donc à organiser spatialement ces valeurs de métrique en fonction de leurs valeurs représentative d'un paramètre de variation de la seconde branche 17 correspondant à des coordonnées axiales. Par exemple, si le paramètre de variation est une position de l'imageur 2, les valeurs de métrique sont organisées en fonction des positions de l'imageur 2 au moment de l'acquisition des images acquises.

La représentation tomographique peut simplement être l'ensemble tridimensionnel formé par la juxtaposition selon l'axe objet Z des images filtrées. Il est préférable toutefois de disposer d'une représentation tomographique bidimensionnelle qui puisse rentre compte en une ou deux dimensions de la structure tridimensionnelle de la zone imagée.

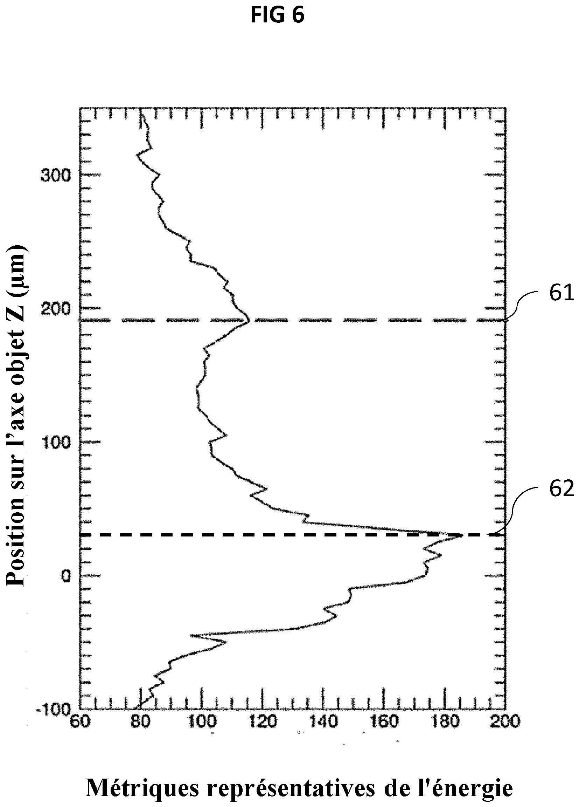

La représentation tomographique peut ainsi être une représentation de type A qui fait correspondre une valeur de métrique représentative de l'énergie à chaque coordonnée axiale. La **figure 6** montre un exemple d'une telle représentation de type A illustrant la coupe d'une rétine selon l'axe objet Z. Sur ce graphique, l'axe des ordonnées correspond aux positions sur l'axe objet Z, en μm, tandis que l'axe des abscisses correspond aux valeurs de métrique représentative de l'énergie. La métrique utilisée est ici à titre d'exemple non limitatif la variance spatiale de l'image. Deux maximums locaux se dégagent sur cette représentation, indiquant deux positions du plan focal objet 21 liées à des couches rétiniennes particulières, qui réfléchissent le mieux le rouge du faisceau lumineux d'éclairage. Ils sont indiqués par les lignes en tirets 61, 62 :

- une première position correspondant à la ligne en tirets 61 se situe environ à 190 μm sur l'axe objet Z, qui correspond à la couche des fibres nerveuses rétiniennes, et
- une seconde position correspondant à la ligne en tirets 62 se situe environ à 30 μm sur l'axe objet Z, qui correspond à la couche des photorécepteurs de l'oeil 3.

On voit donc qu'il est aisé à partir d'une représentation tomographique de type A telle qu'illustrée sur la **figure**

**6** d'identifier les coordonnées axiales de zones particulières. En raison de la correspondance entre la commande du système d'imagerie permettant de modifier la valeur du paramètre de variation de la seconde branche 17 du système optique 14 et les coordonnées axiales des images, on peut déduire les commandes à appliquer au système d'imagerie pour imager une zone précise. Ainsi, le procédé peut comprendre une étape dans laquelle une coordonnée axiale d'un maximum local de la métrique d'énergie est déterminé à partir de la représentation tomographique, et donc une valeur représentative d'un paramètre de variation, et la seconde branche 17 est ensuite réglée en fonction de cette valeur représentative du paramètre de variation pour que le plan focal image 21 ait une position correspondant à une coordonnée axiale du maximum local de la métrique d'énergie, et au moins une image est acquise..

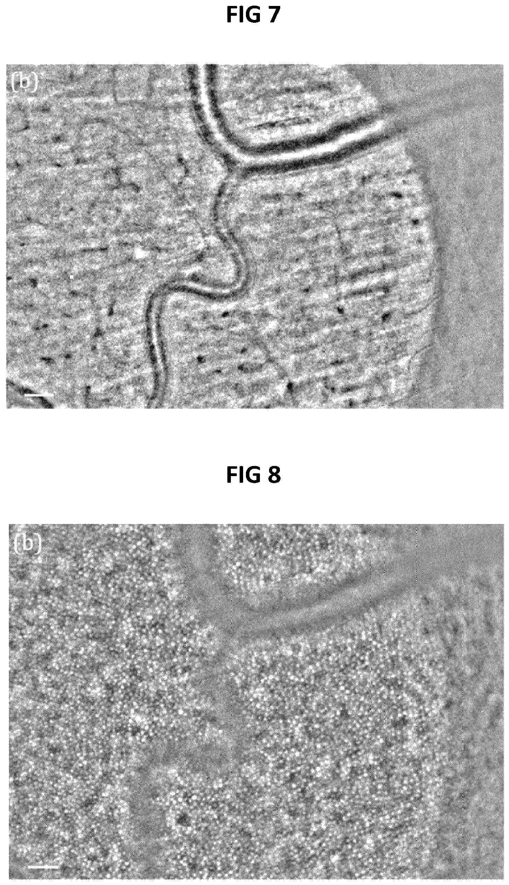

En reprenant l'exemple de la **figure 6,** le système d'imagerie a été configuré en déplaçant l'imageur 2 de sorte que le plan focal objet 20 se trouve à la coordonnée 190 $\mu$m sur l'axe objet Z, et dix images furent acquises successivement. Ces images furent combinées en calculant la moyenne des valeurs de chaque pixel. La **figure** 7 montre le résultat de cette acquisition. On obtient directement une image nette et précisément localisée de la couche de fibres nerveuses de la rétine (l'échelle graphique blanche en bas à gauche est de 50 $\mu$m). De manière similaire, le système d'imagerie a été configuré en déplaçant l'imageur 2 de sorte que le plan focal objet 20 se trouve à la coordonnée 30 $\mu$m sur l'axe objet Z, et dix images furent acquises successivement. Ces images furent combinées en calculant la moyenne des valeurs de chaque pixel. La **figure 8** montre le résultat de cette acquisition. On obtient directement une image nette et précisément localisée de la couche de photorécepteurs (l'échelle graphique blanche en bas à gauche est de 50 $\mu$m).

La représentation tomographique peut ainsi être utilisée pour détecter les couches présentant un contenu important en haute fréquence spatiale, en d'autres termes pour déterminer les plans focaux objets pour lesquels des images nettes et aux caractéristiques significatives peuvent être générées. Cet aspect est d'autant plus intéressant pour l'imagerie ou le traitement de patients présentant des maladies rétiniennes. Alors que pour un sujet sain, les couches réflectives de la rétine sont aisées à localiser, il n'en va pas de même avec une maladie rétinienne, où l'anatomie rétinienne peut complètement changer.

Comme expliqué plus haut, à chaque image est associée une valeur représentative d'un paramètre de variation de la seconde branche 17 du système optique 14 correspondant à la valeur prise par ce paramètre lors de l'acquisition de ladite image. Ces valeurs de paramètres associées aux images acquises sont conservées au cours du procédé, par exemple lors de l'application du filtrage passe-haut, de la détermination de la métrique représentative de l'énergie, ou bien encore lors de la combinaison d'images entre elles.

Il en résulte que l'identification et la localisation d'un élément caractéristique sur la représentation tomographique (comme par exemple une couche particulière) permet de déterminer la coordonnée axiale de cet élément dans la représentation tomographique, et donc d'identifier la valeur représentative d'un paramètre de variation correspondant à l'image où se trouve cette caractéristique. Ce paramètre de variation ainsi déterminé peut être utilisé pour commander le système d'imagerie ou un laser pour viser précisément une zone de l'oeil 3. Dans le cas de l'utilisation de l'imageur 2, il suffit donc de reproduire la variation de la seconde branche 17. Dans le cas de l'utilisation d'un laser, il suffit d'appliquer une variation similaire sur le chemin optique du faisceau laser. Par exemple, si le paramètre de variation associé aux images est une position de l'imageur 2, il suffit d'y faire correspondre une position correspondante du laser, par exemple au moyen d'une relation mathématiques liant une valeur du paramètre de variation à une commande du laser.

La représentation tomographique peut aussi être une représentation de type B. Une telle représentation fait typiquement correspondre une valeur de métrique représentative de l'énergie pour chaque coordonnée axiale et pour chaque coordonnée dans une direction perpendiculaire à l'axe objet Z.

Une telle représentation de type B peut être construite notamment en déterminant une valeur de métrique représentative de l'énergie pour chacune d'une pluralité de zones d'intérêt de chaque image filtrée. Chaque zone d'intérêt présente une première dimension dans une première direction X perpendiculaire à l'axe objet Z, et une seconde dimension dans une seconde direction Y perpendiculaire à l'axe objet Z et à la première direction X. La première dimension est plus faible que la seconde dimension, de préférence d'un facteur 10. De préférence, la seconde dimension recouvre la majorité de la dimension de l'image filtrée dans la seconde direction Y, et de préférence recouvre la totalité de la dimension de l'image filtrée dans la seconde direction Y, typiquement toute sa hauteur. La première dimension peut être réduite à un pixel, ou peut s'étendre sur plusieurs pixels, de préférence toutefois sur un nombre de pixels inférieur à 10. Les zones d'intérêt de l'image filtrée peuvent être distinctes, mais de préférence se recouvrent dans la première direction X, par exemple avec un décalage entre elles dans la première direction X inférieur à leurs largeurs.

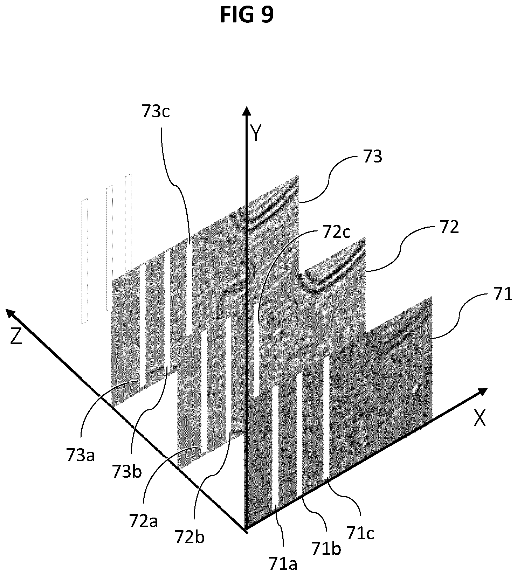

Un exemple simplifié est illustré par la **figure 9,** dans laquelle sont représentées trois images filtrées 71, 72, 73 successives dans la direction de l'axe objet Z. Chaque image filtrée 71, 72, 73 s'étend sur un plan selon la première direction X et la seconde direction Y, qui forment avec la direction de l'axe objet Z un repère orthogonal. Dans chaque image filtrée 71, 72, 73, une première sous-zone 71a, 72a, 73a, est définie, qui s'étend sur toute la hauteur de l'image filtrée dans la seconde direction Y, par exemple sur 80 pixels, mais sur une faible largeur dans la première direction X, par exemple 4 pixels. Une valeur de métrique représentative de l'énergie est calculée pour chaque première sous-zone 71a, 72a, 73a, par exemple l'énergie de fréquence spatiale comme dans le cas illustré, ou bien la variance spatiale. Les positions des sous-zones 71a, 72a, 73a sur les différentes images filtrées restent identiques, correspondant aux mêmes pixels.

Ensuite, une seconde sous-zone 71b, 72b, 73b de même taille que la première sous-zone 71a, 72a, 73a est définie, décalée sur chaque image filtrée 71, 72, 73 dans la première direction X par rapport à la première sous-zone 71a, 72a, 73a. A des fins de clarification, la seconde sous-zone 71b, 72b, 73b est montrée sur la **figure 9** comme ne recouvrant pas la première sous-zone 71a, 72a, 73a. Dans les faits, la seconde sous-zone 71b, 72b, 73b est décalée dans la première direction X d'une distance inférieur à la largeur de la première sous-zone 71a, 72a, 73a, et recouvre donc celle-ci. Par exemple, avec des sous-zones de 4 pixels de large, le décalage peut n'être que d'un pixel entre deux sous-zones. De même que précédemment, une valeur de métrique représentative de l'énergie est calculée pour chaque seconde sous-zone 71b, 72b, 73b. Puis il en va de même pour des troisièmes sous-zones 71c, 72c, 73c décalée par rapport aux secondes sous-zones 71b, 72b, 73b.

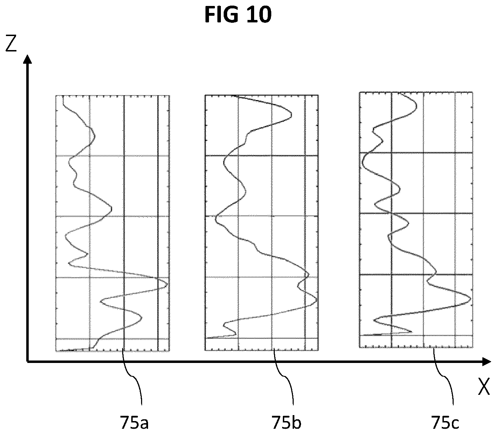

Il en résulte pour une pluralité de coordonnées selon la première direction X, une évolution de la valeur de métrique représentative de l'énergie dans la direction de l'axe objet Z. La **figure 10** montre un exemple schématique de ce résultat, avec trois courbes 75a, 75b, 75c représentant l'évolution de la valeur de métrique représentative de l'énergie spatiale dans la direction de l'axe objet Z pour les positions dans la première direction X correspondant aux trois sous-zones précédemment mentionnées.

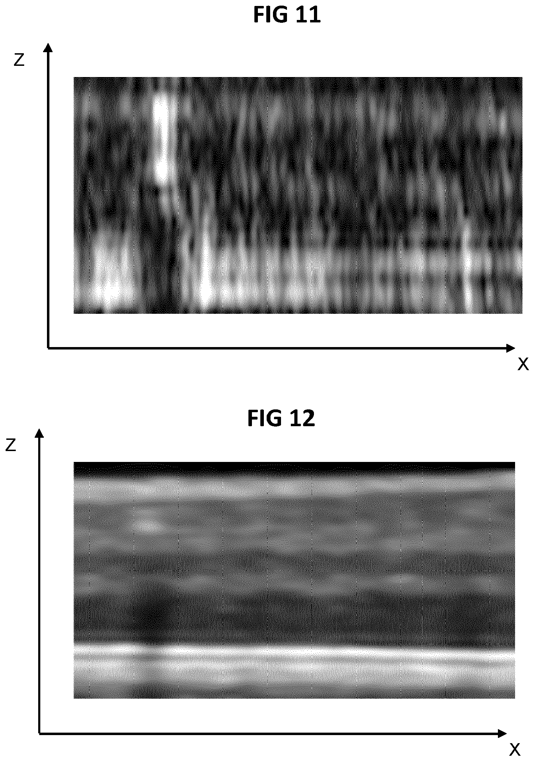

En traduisant en valeur de pixel, par exemple en niveau de gris, cette évolution de la valeur de métrique représentative de l'énergie dans la direction de l'axe objet Z, il est possible de construire une image constituant la représentation tomographique de type B, ainsi qu'illustré sur la **figure 11.** En comparaison de la même zone de la rétine de l'oeil 3 imagée par OCT illustrée par la **figure 12,** on constate que la représentation tomographique selon l'invention arrive à un résultat très satisfaisant, sans nécessiter la mise en oeuvre de complexes mesures d'interférences. En outre, contrairement à la technique OCT, la profondeur (selon Z) dans la rétine de chaque couche est connue, ainsi que les commandes du système d'imagerie correspondantes, comme montré plus haut.

La taille des sous-zones influe le contraste et la résolution de la représentation tomographique. Plus la tailles des sous-zones est importante, moins les détails seront conservés (comme les vaisseaux sanguins longitudinaux et les photorécepteurs), et les couches seront plus lisses, comme dans le cas de l'image OCT de la **figure 12.** En réduisant la taille des sous-zones, la représentation tomographique est plus bruitée, mais les détails plus fins sont conservés, permettant de mieux visualiser les structures telles que les vaisseaux sanguins ou les photorécepteurs.

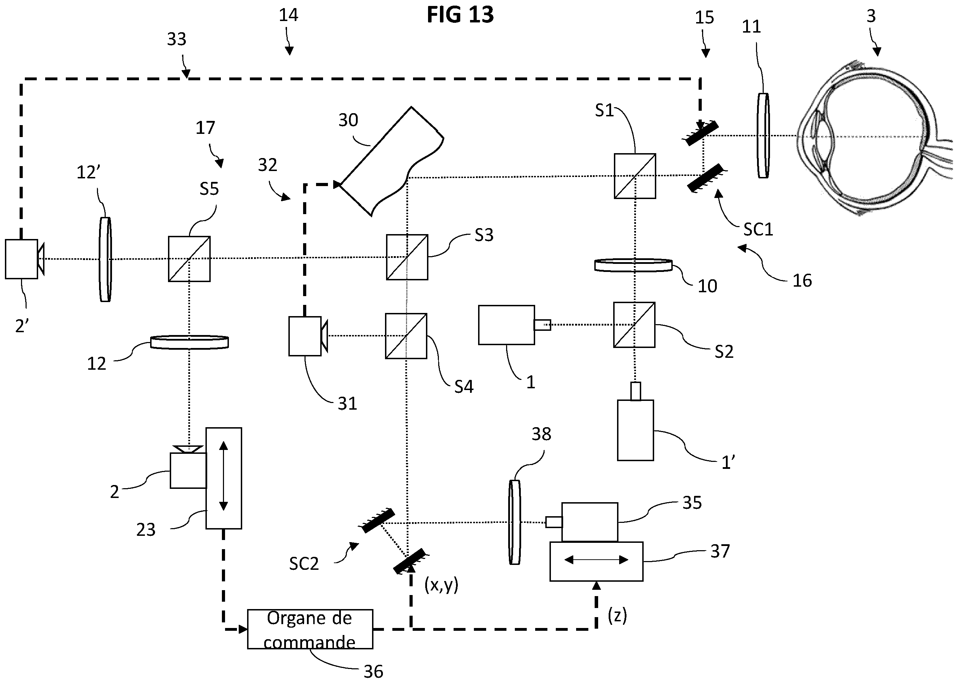

Le système illustré par la **figure 1** est un schéma simplifié d'un exemple de système d'imagerie selon un mode de réalisation possible de l'invention. Comme indiqué plus haut, le système optique 14 comprend typiquement de nombreux éléments optiques. En particulier, le système optique 14 peut comprendre des éléments d'une boucle d'optique adaptative et des éléments de déplacement des faisceaux lumineux. En outre, le système d'imagerie peut comprendre un laser de traitement. La **figure 13** présente ainsi un exemple plus détaillé de système d'imagerie, à titre d'exemple non limitatif, les différents éléments le constituant pouvant être combinés où être pris isolément dans un système d'imagerie.

La lumière émise par la source lumineuse 1, dite d'illumination passe par une séparatrice S2 qu'elle partage avec une seconde source lumineuse 1' dont le faisceau est assimilable à celui émis par une source ponctuelle, de faible étendue. Cette seconde source lumineuse 1' est utilisée pour détecter ces altérations du front d'onde.

En effet, un oeil 3 humain réel n'est pas strictement stigmatique, c'est-à-dire que l'image d'un point n'est pas un point rigoureusement net. La propagation de la lumière dans l'oeil 3 met en évidence des aberrations oculaires qui font naître des défauts dans cette lumière, et en particulier en ce qui concerne son front d'onde. Ces aberrations oculaires peuvent être statiques, les exemples les plus courants étant les défauts de vision corrigés par les lunettes (myopie, hypermétropie, astigmatisme, etc.). Les aberrations peuvent également être dynamiques, par exemple causées par des micro-accommodations du cristallin, l'écoulement du film lacrymal, et les mouvements oculaires. Ces aberrations se traduisent par une fonction d'étalement du point ("Point Spread Function" ou PSF en anglais) qui s'éloigne de celle d'un oeil parfait théorique, et qui varie rapidement dans le temps. La présence d'une optique adaptative permet de corriger ces défauts de font d'onde. De préférence, la seconde source lumineuse présente des longueurs d'onde comprise entre 600 nm et 700 nm, notamment car cette plage met en valeur les aberrations principales, et est de préférence encore monochromatique.

La lumière sortant de la séparatrice S2 traverse ensuite l'entrée 10, et rejoint par la séparatrice S1 la partie commune 15 du système optique 14 en direction de l'oeil 3. Un premier actionneur permet de commander le déplacement du faisceau lumineux dans le chemin optique par rapport à la rétine de l'oeil 3. Le premier actionneur comprend un scanner SC1 formé deux miroirs pivotants, et permet de balayer toute la zone centrale de la rétine.

La lumière traverse ensuite une interface 11 face à l'oeil 3 par laquelle sort le faisceau lumineux d'éclairage pour atteindre l'oeil 3. Le faisceau lumineux d'éclairage traverse la pupille, le corps vitré, et atteint la rétine 6. La lumière rencontre les différents tissus constituant la rétine 6, organisés en couches rétiniennes à différentes profondeurs. La lumière est rétrodiffusée par ces tissus et ressort de l'oeil, constituant un faisceau lumineux d'image en provenance de l'oeil qui entre dans le système optique 14 par l'interface 11. Le faisceau lumineux d'image repasse par le scanner SC1,

EP 3 958 728 B1

puis traverse la séparatrice S1, et atteint un élément correcteur 30 de l'optique adaptative, utilisé pour corriger le front d'onde des faisceaux lumineux traversant l'oeil 3, aussi bien le faisceau lumineux d'image que le faisceau laser, afin de compenser les déformations subies par les flux lumineux dans l'oeil 3. Typiquement, l'élément correcteur 30 est un miroir déformable dont la surface se modifie pour compenser les déformations de front d'onde mesurées.

Le flux lumineux d'image passe ensuite par une séparatrice S3 qui dérive du chemin optique une image de la rétine vers un ou plusieurs imageurs, en amont de l'élément correcteur 30 de l'optique adaptative. Une autre partie du flux lumineux d'image continue vers une séparatrice S4 qui dévie le flux lumineux vers un analyseur de surface d'onde 31 ou analyseur de front d'onde, configuré pour recevoir de la lumière du chemin optique. Dans le cas illustré, la lumière est dérivée au moyen de la séparatrice S4 placée sur le chemin optique. L'analyseur de surface d'onde 31 détermine une mesure représentative d'une forme d'une surface d'onde de la lumière transitant le long du chemin optique. Plus précisément, l'analyseur de surface d'onde 31 décompose un front d'onde en fronts d'ondes élémentaires et il permet de déterminer pour chaque front d'onde élémentaire son orientation. La mesure de ces orientations permet après intégration de remonter à la forme du front d'onde.

Une boucle de retour 32 permet d'utiliser les mesures de l'analyseur de surface d'onde 31 pour commander l'élément correcteur 30. Plus précisément, les mesures de l'analyseur de surface d'onde 31 sont reçues et traitées par un module de traitement de la boucle de retour 32, de préférence un calculateur temps réel, configuré pour recevoir la mesure de l'analyseur de surface d'onde, et commander l'élément correcteur 30 en fonction de la mesure de l'analyseur de surface d'onde, afin de compenser les perturbations détectées. Dans le cas typique où l'élément correcteur 30 est un miroir déformable, le module de traitement calcule les commandes (par exemple tensions ou intensités) à envoyer au miroir déformable, également placé dans un plan pupille. La surface du miroir déformable se modifie alors pour compenser les déformations de front d'onde mesurées.

Le flux lumineux d'image dérivé par la séparatrice S3 à destination de l'imageur 2 passe ensuite dans une séparatrice S5 qui permet de dériver une partie du flux lumineux parcourant le chemin optique vers la sortie 12 face à l'imageur 2, par laquelle sort le faisceau lumineux d'image pour atteindre l'imageur 2, ce qui permet d'obtenir une image de la zone de la rétine de l'oeil 3 faisant face à l'interface 11.

Un imageur secondaire 2' peut être prévu, alimenté en flux lumineux à partir de la séparatrice S5 au travers d'une sortie secondaire 12'. Cet imageur secondaire 2' est une caméra de stabilisation est utilisée pour mettre en oeuvre une régulation pour asservir en position l'interface 11 vis-à-vis de la rétine à traiter. Une boucle de stabilisation 33 relie l'imageur secondaire 2' au premier actionneur. Plus précisément, l'imageur secondaire 2' transmet des images acquises à un module de traitement de la boucle de stabilisation 33, de préférence un calculateur temps réel, qui à partir de ces images détermine une commande du scanner SC1 du premier actionneur pour déplacer le faisceau lumineux dans le chemin optique par rapport à la rétine de l'oeil 3 afin de stabiliser la position du faisceau lumineux, notamment en compensant les mouvements involontaires de l'oeil 3. Le module de traitement peut par exemple exploiter une image des photorécepteurs et un algorithme de type corrélation pour déterminer les mouvements de la rétine, et déterminer une commande du premier actionneur pour que le faisceau lumineux suive ces mouvements.

Le système peut comprendre un laser de photocoagulation 35 configuré pour émettre dans une direction d'émission un faisceau laser dans le système optique. Le laser de photocoagulation 35 peut comprendre une source laser et une fibre optique de transport présentant un diamètre de coeur de préférence inférieur à 12 μm, et de préférence encore inférieur à 8 μm. La source laser est de préférence une source laser à fibre, c'est-à-dire dont le milieu amplificateur est créé dans une fibre optique dopée par une terre rare, et peut être monomode. Le laser de photocoagulation 35 est configuré pour émettre dans une longueur d'onde comprise entre 520 nm et 690 nm, de préférence entre 540 nm et 630 nm.

Le laser de photocoagulation 35 peut être associé à un organe de commande 36 configuré pour déterminer la valeur du paramètre de variation correspondant à la position d'une cible dans la représentation tomographique dérivée des images acquises par l'imageur 2, et pour commander le laser de photocoagulation 35 au moyen d'une commande de profondeur (z) dérivée de cette valeur du paramètre de variation. Plus précisément, le laser de photocoagulation 35 est mobile en translation dans sa direction d'émission et la commande de profondeur est une commande de déplacement du laser de photocoagulation 35. A cet effet, un moteur 37 peut être prévu pour déplacer en translation le laser de photocoagulation 35 dans sa direction d'émission. C'est ce moteur qui reçoit alors la commande de profondeur (z) envoyée par l'organe de commande 36.

Puisque la représentation tomographique associe des valeurs de paramètre de variation à chaque zone de la zone imagée (comme par exemple une position de l'imageur 2), il est possible d'attribuer une valeur de paramètre de variation d'une cible laser identifiée sur la représentation tomographique. L'organe de commande 36 peut donc déterminer les commandes à appliquer au laser de photocoagulation 35 pour que le faisceau laser atteigne cette cible laser de façon précise, sans endommager les tissus adjacents, par exemple au moyen d'une relation liant une valeur du paramètre de variation à une valeur de commande du laser, comme par exemple un déplacement du laser ou une position du laser.

Il est possible qu'un étalonnage soit nécessaire afin d'établir la relation liant une valeur du paramètre de variation à une valeur de commande du laser, de façon aisée pour l'homme du métier via quelques tests. Il faut toutefois prendre

14

en compte une possible différence d'aberration chromatique longitudinale résultant de l'emploi de longueurs d'onde différentes pour le faisceau lumineux d'illumination et le faisceau laser.

L'organe de commande 36 est en outre configuré pour déterminer une commande de déplacement (x, y) du faisceau laser au moins dans un plan perpendiculaire à la direction de propagation du laser, défini par deux directions distinctes notées x et y. Pour ce faire, le second actionneur peut comprendre, ainsi qu'illustré sur la **figure 13,** un scanner SC2 positionné dans le chemin optique et adapté pour réaliser un déplacement du faisceau laser dans un plan focal (x, y) perpendiculaire à la propagation du faisceau laser, Le scanner SC2 agit par exemple en modifiant les orientations respectives de deux miroirs se faisant face, décalant ainsi angulairement le faisceau laser qui en ressort.

L'organe de commande 36 peut être tout ordinateur, comme par exemple un calculateur temps-réel, comprenant un processeur et une mémoire, apte à déterminer des commandes pour le faisceau laser à partir d'informations dérivées de la représentation tomographique de l'oeil 3, éventuellement complétées par d'autres informations, dont certaines peuvent correspondre à des données entrées par un opérateur, comme par exemple une consigne de zone à traiter.

Après avoir été positionné le long de sa direction par le moteur 37 commandé par la commande de profondeur (z) envoyée par l'organe de commande 36, le laser de photocoagulation 35 émet un émet un faisceau laser traversant la lentille 38, puis arrive sur le scanner SC2 où il est réorienté selon les directions x et y conformément aux commandes de déplacement envoyées par l'organe de commande 36. Le faisceau laser rejoint ensuite la séparatrice S4, puis parcourt le même chemin optique (dans le sens inverse) que le faisceau lumineux d'image, à savoir qu'il passe par la séparatrice S3, l'élément correcteur 30 de l'optique adaptative, la séparatrice S1, et le premier actionneur (scanner SC1) avant de traverser l'interface 11 pour atteindre la rétine de l'oeil 3.

L'invention n'est pas limitée au mode de réalisation décrit et représenté aux figures annexées. Des modifications restent possibles, notamment du point de vue de la constitution des divers éléments ou par substitution d'équivalents techniques, sans sortir pour autant du domaine de protection de l'invention.

## Revendications

1. Procédé d'imagerie ophtalmique d'une partie à imager d'un oeil au moyen d'un système d'imagerie ophtalmique, ledit système d'imagerie ophtalmique comprenant :

   - une source lumineuse (1) adaptée pour émettre un faisceau lumineux d'éclairage,
   - un imageur (2) adapté pour acquérir une image d'un faisceau lumineux renvoyé,
   - un système optique (14) adapté pour la propagation de faisceaux lumineux, le système optique (14) comprenant une partie commune (15) s'étendant jusqu'à une interface (11) face à l'oeil (3), une première branche (16) reliant la source lumineuse à la partie commune (15), et une seconde branche (17) reliant l'imageur (2) à la partie commune (15), **caractérisé en ce que** le procédé comprend les étapes suivantes :

      a) éclairage de la partie à imager de l'oeil par un faisceau lumineux d'éclairage émis par la source lumineuse (1), et acquisition par l'imageur (2) d'une série d'images de la zone à imager correspondants à une pluralité de plans focaux objet (20) différents distribués le long d'un axe objet (Z), l'acquisition comprenant une modification d'un paramètre de variation de la seconde branche (17) du système optique (14) entre chaque image acquise,
      b) application d'un filtrage passe-haut en fréquences spatiales sur chacune des images de la série d'image pour obtenir une série d'images filtrées,
      c) pour chaque image filtrée, détermination d'au moins une valeur de métrique représentative de l'énergie d'une zone d'intérêt de chaque image filtrée, et
      d) combinaison de valeurs d'une métrique représentative de l'énergie de différentes images filtrées, chacune associée avec des coordonnées axiales représentatives de la distribution des plans focaux objet (20) le long de l'axe objet (Z), pour obtenir une représentation tomographique.

2. Procédé selon la revendication 1, dans lequel les coordonnées axiales correspondent à des valeurs représentatives d'un paramètre de variation de la seconde branche (17) des images acquises correspondant aux images filtrées.

3. Procédé selon l'une des revendications précédentes, dans lequel la modification de la seconde branche du système optique entre chaque image acquise comprend une translation de l'imageur, et/ou une modification d'un élément optique présent dans la seconde branche du système optique.

4. Procédé selon l'une des revendications précédentes, dans lequel la série d'images de la rétine comprend au moins 20 images acquises sans modification de la première branche et de la partie commune du système optique.

**5.** Procédé selon l'une des revendications précédentes, dans lequel l'acquisition de la série d'images est effectuée en moins de 5 secondes.

**6.** Procédé selon l'une des revendications précédentes, dans lequel la représentation tomographique est une représentation de type A qui fait correspondre une métrique représentative de l'énergie pour une pluralité de coordonnées axiales, ou la représentation tomographique est une représentation de type B qui fait correspondre une métrique représentative de l'énergie pour une pluralité de coordonnées axiales et pour une pluralité de coordonnées dans une direction perpendiculaire à l'axe objet.

**7.** Procédé selon l'une des revendications précédentes, dans lequel des images acquises successives sont combinées entre elles pour résulter en une image combinée dont les valeurs de pixels correspondent à une combinaison des valeurs des pixels des images acquises successives.

**8.** Procédé selon la revendication précédente, dans lequel au moins trois images acquises successives sont combinées entre elles, et la combinaison des valeurs des pixels des images acquises successives consiste en une moyenne de ces valeurs des pixels des images acquises successives.

**9.** Procédé selon l'une quelconque des revendications précédentes, dans lequel la métrique représentative de l'énergie est une énergie de fréquence spatiale, une variance spatiale de l'image, une énergie laplacienne de l'image, ou une fonction de Brenner de l'image.

**10.** Procédé selon l'une quelconque des revendications précédentes, comprenant en outre une étape e) dans laquelle une valeur représentative d'un paramètre de variation de la seconde branche (17) correspondant à un maximum local de la métrique d'énergie est déterminé à partir de la représentation, et la seconde branche (17) est ensuite réglée selon cette valeur représentative du paramètre de variation de sorte que le plan focal image (21) ait une position correspondant à une coordonnée axiale du maximum local de la métrique d'énergie, et au moins une image est acquise.

**11.** Système d'imagerie ophtalmique comprenant :

- une source lumineuse (1) adaptée pour émettre un faisceau lumineux d'éclairage,
- un imageur (2) adapté pour acquérir une image d'un faisceau lumineux renvoyé,
- un système optique (14) adapté pour la propagation de faisceaux lumineux, le système optique (14) comprenant une partie commune (15) s'étendant jusqu'à une interface (11) face à l'oeil (3), une première branche (16) reliant la source lumineuse à la partie commune (15), et une seconde branche (17) reliant l'imageur (2) à la partie commune (15), le système d'imagerie ophtalmique étant configuré pour:

a) éclairer la partie à imager de l'oeil par un faisceau lumineux d'éclairage émis par la source lumineuse (1), et acquérir par l'imageur (2) une série d'images de la zone à imager correspondants à une pluralité de plans focaux objet (20) différents distribués le long d'un axe objet (Z), l'acquisition comprenant une modification de la seconde branche (17) du système optique (14) entre chaque image acquise,

b) appliquer un filtrage passe-haut en fréquences spatiales sur chacune des images de la série d'image pour obtenir une série d'images filtrées,

c) pour chaque image filtrée, déterminer au moins une métrique représentative de l'énergie d'une zone d'intérêt de chaque image filtrée, et

d) combiner des valeurs d'une métrique représentative de l'énergie de différentes images filtrées, chacune associée avec des coordonnées axiales représentatives de la distribution des plans focaux objet (20) le long de l'axe objet (Z), pour obtenir une représentation tomographique,

le système d'imagerie ophtalmique étant configuré pour mettre en oeuvre le procédé selon l'une quelconque des revendications précédentes.

**12.** Système de photocoagulation laser d'une rétine, comprenant :

- un système d'imagerie ophtalmique selon la revendication 11,
- un laser de photocoagulation (35) configuré pour émettre dans une direction d'émission un faisceau laser dans le système optique (14),

le système de photocoagulation laser comprenant un organe de commande du laser de photocoagulation, l'organe de commande étant configuré pour déterminer, à partir de la représentation tomographique, une valeur représentative d'un paramètre de variation de la seconde branche (17) correspondant à une coordonnée axiale d'une cible selon l'axe objet (Z) dans la représentation tomographique, et pour commander le laser de photocoagulation au moyen d'une commande de profondeur dérivée de cette valeur représentative d'un paramètre de variation.

13. Système selon la revendication précédente, dans lequel le laser est mobile en translation dans sa direction d'émission et la commande de profondeur est une commande de déplacement du laser.

**Patentansprüche**

1. Ophthalmisches Bildgebungsverfahren eines abzubildenden Teils eines Auges mittels eines ophthalmischen Bildgebungssystems, wobei das ophthalmische Bildgebungssystem umfasst:

   - eine Lichtquelle (1), die geeignet ist, einen Beleuchtungslichtstrahl zu senden,
   - einen Bildgeber (2), der geeignet ist, ein Bild eines zurückgesendeten Lichtstrahls zu erfassen,
   - ein für die Ausbreitung von Lichtstrahlen geeignetes optisches System (14), wobei das optische System (14) einen gemeinsamen Teil (15) umfasst, der sich bis zu einer Schnittstelle (11) am Auge (3) erstreckt, wobei ein erster Arm (16) die Lichtquelle mit dem gemeinsamen Teil (15) verbindet und ein zweiter Arm (17) den Bildgeber (2) mit dem gemeinsamen Teil (15) verbindet, **dadurch gekennzeichnet, dass** das Verfahren die folgenden Schritte umfasst

   a) Beleuchten des abzubildenden Teils des Auges durch einen von der Lichtquelle (1) gesendeten Beleuchtungslichtstrahl und Erfassen durch den Bildgeber (2) einer Reihe von Bildern des abzubildenden Bereichs, die einer Vielzahl unterschiedlicher Objektfokalebenen (20) entsprechen, die entlang einer Objektachse (Z) verteilt sind, wobei die Erfassung eine Änderung eines Variationsparameters des zweiten Arms (17) des optischen Systems (14) zwischen jedem erfassten Bild umfasst,
   b) Anwenden eines Hochpassfilters in räumlichen Frequenzen auf jedes der Bilder der Bildserie, um eine Serie gefilterter Bilder zu erhalten,
   c) Bestimmen, für jedes gefilterte Bild, mindestens eines Metrikwerts, der für die Energie eines interessierenden Bereichs jedes gefilterten Bildes repräsentativ ist, und
   d) Kombinieren von Werten einer Metrik, die für die Energie verschiedener gefilterter Bilder repräsentativ ist, die jeweils axialen Koordinaten zugeordnet sind, die für die Verteilung der Objektfokalebenen (20) entlang der Objektachse (Z) repräsentativ sind, um eine tomografische Darstellung zu erhalten.

2. Verfahren nach Anspruch 1, wobei die Achskoordinaten repräsentativen Werten eines Variationsparameters des zweiten Arms (17) der erfassten Bilder entsprechen, die den gefilterten Bildern entsprechen.

3. Verfahren nach einem der vorhergehenden Ansprüche, wobei die Änderung des zweiten Arms des optischen Systems zwischen jedem erfassten Bild eine Verschiebung des Bildgebers und/oder eine Änderung eines im zweiten Arm des optischen Systems vorhandenen optischen Elements umfasst.

4. Verfahren nach einem der vorhergehenden Ansprüche, wobei die Bildserie der Netzhaut mindestens 20 Bilder umfasst, die ohne Änderung des ersten Arms und des gemeinsamen Teils des optischen Systems erfasst wurden.

5. Verfahren nach einem der vorhergehenden Ansprüche, wobei die Erfassung der Bildserie in weniger als 5 Sekunden erfolgt.

6. Verfahren nach einem der vorhergehenden Ansprüche, wobei die tomografische Darstellung eine Darstellung des Typs A ist, die eine Metrik, die für die Energie für eine Vielzahl von Axialkoordinaten repräsentativ ist, in Übereinstimmung bringt, oder die tomografische Darstellung eine Darstellung des Typs B ist, die eine Metrik, die für die Energie für eine Vielzahl von Axialkoordinaten und eine Vielzahl von Koordinaten in einer Richtung senkrecht zur Objektachse repräsentativ ist, in Übereinstimmung bringt.

7. Verfahren nach einem der vorhergehenden Ansprüche, wobei aufeinanderfolgenden erfassten Bilder miteinander kombiniert werden, um ein kombiniertes Bild zu erhalten, dessen Pixelwerte einer Kombination der Pixelwerte der aufeinanderfolgenden erfassten Bilder entsprechen.

8. Verfahren nach vorhergehendem Anspruch, wobei wenigstens drei aufeinanderfolgende erfasste Bilder miteinander kombiniert werden, und die Kombination der Pixelwerte der aufeinanderfolgenden erfassten Bilder aus einem Durchschnitt dieser Pixelwerte der aufeinanderfolgenden erfassten Bilder besteht.

9. Verfahren nach einem der vorhergehenden Ansprüche, wobei die Metrik, die für die Energie repräsentativ ist, eine räumliche Frequenzenergie, eine räumliche Varianz des Bildes, eine laplazische Energie des Bildes oder eine Brennerfunktion des Bildes ist.

10. Verfahren nach einem der vorhergehenden Ansprüche, das ferner einen Schritt e) umfasst, bei dem ein Wert, der für einen Variationsparameter des zweiten Arms (17) repräsentativ ist, der einem lokalen Maximum der Energiemetrik entspricht, aus der Darstellung bestimmt wird, und der zweite Arm (17) dann gemäß diesem repräsentativen Wert des Variationsparameters so eingestellt wird, dass die Bildfokusebene (21) eine Position hat, die einer axialen Koordinate des lokalen Maximums der Energiemetrik entspricht, und mindestens ein Bild erfasst wird.

11. Ophthalmisches Bildgebungssystem, umfassend:

- eine Lichtquelle (1), die geeignet ist, einen Beleuchtungslichtstrahl zu senden,
- einen Bildgeber (2), der geeignet ist, ein Bild eines zurückgesendeten Lichtstrahls zu erfassen,
- ein für die Ausbreitung von Lichtstrahlen geeignetes optisches System (14), wobei das optische System (14) einen gemeinsamen Teil (15) umfasst, der sich bis zu einer Schnittstelle (11) am Auge (3) erstreckt, wobei ein erster Arm (16) die Lichtquelle mit dem gemeinsamen Teil (15) verbindet und ein zweiter Arm (17) den Bildgeber (2) mit dem gemeinsamen Teil (15) verbindet, wobei das ophthalmische Bildgebungssystem ausgelegt ist, um:

a) den abzubildenden Teil des Auges durch einen von der Lichtquelle (1) gesendeten Beleuchtungslichtstrahl zu beleuchten und durch den Bildgeber (2) eine Reihe von Bildern des abzubildenden Bereichs zu erfassen, die einer Vielzahl unterschiedlicher Objektfokalebenen (20) entsprechen, die entlang einer Objektachse (Z) verteilt sind, wobei die Erfassung eine Änderung des zweiten Arms (17) des optischen Systems (14) zwischen jedem erfassten Bild umfasst,
b) einen Hochpassfilter in räumlichen Frequenzen auf jedes der Bilder der Bildserie anzuwenden, um eine Serie gefilterter Bilder zu erhalten,
c) für jedes gefilterte Bild mindestens eine Metrik zu bestimmen, die für die Energie eines interessierenden Bereichs jedes gefilterten Bildes repräsentativ ist, und
d) Werte einer Metrik zu kombinieren, die für die Energie verschiedener gefilterter Bilder repräsentativ ist, die jeweils axialen Koordinaten zugeordnet sind, die für die Verteilung der Objektfokalebenen (20) entlang der Objektachse (Z) repräsentativ sind, um eine tomografische Darstellung zu erhalten,

wobei das ophthalmische Bildgebungssystem ausgelegt ist, um das Verfahren nach einem der vorhergehenden Ansprüche durchzuführen.

12. Laserphotokoagulationssystem für eine Netzhaut, umfassend:

- ein ophthalmisches Bildgebungssystem nach Anspruch 11,
- einen Photokoagulationslaser (35), der ausgelegt ist, um in einer Senderichtung einen Laserstrahl in das optische System (14) zu senden,

wobei das Laserphotokoagulationssystem ein Steuerorgan des Photokoagulationslasers umfasst, wobei das Steuerorgan ausgelegt ist, um aus der tomografischen Darstellung einen repräsentativen Wert eines Variationsparameters des zweiten Arms (17) zu bestimmen, der einer axialen Koordinate eines Ziels gemäß der Objektachse (Z) in der tomografischen Darstellung entspricht, und um den Photokoagulationslaser mithilfe einer Tiefensteuerung, die von diesem Wert abgeleitet wird, der für einen Variationsparameter repräsentativ ist, zu steuern.

13. System nach vorhergehendem Anspruch, wobei der Laser in seiner Senderichtung translatorisch beweglich ist und die Tiefensteuerung eine Bewegungssteuerung des Lasers ist.

**Claims**

1. A method of ophthalmic imaging of a portion to be imaged of an eye by means of an ophthalmic imaging system, said ophthalmic imaging system comprising :

- a light source (1) adapted to emit an illuminating light beam,
- an imager (2) adapted to acquire an image of a returned light beam,
- an optical system (14) adapted for light beam propagation, the optical system (14) comprising a common portion (15) extending to an interface (11) facing the eye (3), a first branch (16) connecting the light source to the common portion (15), and a second branch (17) connecting the imager (2) to the common portion (15),

**characterized in that** the method comprises the following steps:

a) illumination of the part of the eye to be imaged by an illuminating light beam emitted by the light source (1), and acquisition by the imager (2) of a series of images of the area to be imaged corresponding to a plurality of different object focal planes (20) distributed along an object axis (Z), the acquisition comprising a modification of a variation parameter of the second branch (17) of the optical system (14) between each image acquired,
b) applying high-pass spatial frequency filtering to each of the images in the image series to obtain a series of filtered images,
c) for each filtered image, determining at least one metric value representative of the energy of an area of interest in each filtered image, and
d) combining values of a metric representative of the energy of different filtered images, each associated with axial coordinates representative of the distribution of object focal planes (20) along the object axis (Z), to obtain a tomographic representation.

2. Method according to claim 1, in which the axial coordinates correspond to values representative of a parameter of variation of the second branch (17) of the acquired images corresponding to the filtered images.

3. A method according to one of the preceding claims, wherein the modification of the second branch of the optical system between each acquired image comprises a translation of the imager, and/or a modification of an optical element present in the second branch of the optical system.

4. A method according to one of the preceding claims, wherein the series of retinal images comprises at least 20 images acquired without modification of the first branch and the common part of the optical system.

5. Method according to one of the preceding claims, in which the acquisition of the series of images is carried out in less than 5 seconds.

6. A method according to one of the preceding claims, in which the tomographic representation is a type A representation which matches a metric representative of energy for a plurality of axial coordinates, or the tomographic representation is a type B representation which matches a metric representative of energy for a plurality of axial coordinates and for a plurality of coordinates in a direction perpendicular to the object axis.

7. A method according to one of the preceding claims, in which successive acquired images are combined together to result in a combined image whose pixel values correspond to a combination of the pixel values of successive acquired images.

8. A method according to the preceding claim, in which at least three successive acquired images are combined together, and the combination of the pixel values of the successive acquired images consists of an average of these pixel values of the successive acquired images.

9. A method according to any one of the preceding claims, wherein the metric representative of the energy is a spatial frequency energy, a spatial variance of the image, a Laplacian energy of the image, or a Brenner function of the image.

10. A method according to any one of the preceding claims, further comprising a step e) in which a value representative of a variation parameter of the second branch (17) corresponding to a local maximum of the energy metric is determined from the representation, and the second branch (17) is then adjusted according to this representative value of the variation parameter so that the image focal plane (21) has a position corresponding to an axial coordinate of the local maximum of the energy metric, and at least one image is acquired.

11. An ophthalmic imaging system comprising :

- a light source (1) adapted to emit an illuminating light beam,
- an imager (2) adapted to acquire an image of a returned light beam,
- an optical system (14) adapted for light beam propagation, the optical system (14) comprising a common portion (15) extending to an interface (11) facing the eye (3), a first branch (16) connecting the light source to the common portion (15), and a second branch (17) connecting the imager (2) to the common portion (15), the ophthalmic imaging system being configured to:

a) illuminate the part of the eye to be imaged by an illuminating light beam emitted by the light source (1), and acquire by the imager (2) a series of images of the area to be imaged corresponding to a plurality of different object focal planes (20) distributed along an object axis (Z), the acquisition comprising a modification of the second branch (17) of the optical system (14) between each acquired image,

b) applying high-pass spatial frequency filtering to each of the images in the image series to obtain a series of filtered images,

c) for each filtered image, determine at least one metric representative of the energy of an area of interest in each filtered image, and

d) combine values of a metric representative of the energy of different filtered images, each associated with axial coordinates representative of the distribution of object focal planes (20) along the object axis (Z), to obtain a tomographic representation,

the ophthalmic imaging system being configured to implement the method according to any one of the preceding claims.

12. A system for laser photocoagulation of a retina, comprising :

- an ophthalmic imaging system according to claim 11,
- a photocoagulation laser (35) configured to emit a laser beam in an emission direction into the optical system (14),

the laser photocoagulation system comprising a controller for the photocoagulation laser, the controller being configured to determine, from the tomographic representation, a value representative of a parameter of variation of the second branch (17) corresponding to an axial coordinate of a target along the object axis (Z) in the tomographic representation, and to control the photocoagulation laser by means of a depth command derived from this value representative of a parameter of variation.

13. A system according to the preceding claim, in which the laser is translatable in its emission direction and the depth control is a laser displacement control.

# FIG 1

Axe objet Z

## FIG 2

## FIG 3

## FIG 4a

## FIG 4b

# FIG 5a

# FIG 5b

# FIG 6

Axis label (vertical): Position sur l'axe objet Z (µm)

Axis label (horizontal): Métriques représentatives de l'énergie

## FIG 7

## FIG 8

**FIG 9**

# FIG 10

75a      75b      75c

# FIG 11

# FIG 12

**FIG 13**

EP 3 958 728 B1

## RÉFÉRENCES CITÉES DANS LA DESCRIPTION

**Documents brevets cités dans la description**

- US 2014268049 A

- US 2013063698 A